(19)

**Europäisches Patentamt**
**European Patent Office**
**Office européen des brevets**

(11) **EP 4 749 280 A1**

(12) **EUROPEAN PATENT APPLICATION**

(43) Date of publication:
**27.05.2026 Bulletin 2026/22**

(21) Application number: **25172340.9**

(22) Date of filing: **24.04.2025**

(51) International Patent Classification (IPC):
**G01N 33/50** (2006.01)  **G01N 33/569** (2006.01)
**G01N 33/574** (2006.01)

(52) Cooperative Patent Classification (CPC):
**G01N 33/56972; G01N 33/505; G01N 33/575;**
G01N 2333/70517; G01N 2333/70596;
G01N 2800/52; G01N 2800/7042

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **22.11.2024 EP 24306949**

(71) Applicant: **Assistance Publique - Hôpitaux de
Paris
75012 Paris (FR)**

(72) Inventors:
• **GRANIER, Clémence**
**75015 Paris (FR)**
• **PAILLAUD, Elena**
**92190 Meudon (FR)**
• **GONNIN, Cécile**
**78000 Versailles (FR)**

(74) Representative: **Novagraaf Technologies
2 rue Sarah Bernhardt
CS90017
92665 Asnières-sur-Seine Cedex (FR)**

(54) **METHOD FOR DETERMINING THE SENSITIVITY OF A CANCER TO PD-1/PD-L1 THERAPY**

(57) The present invention belongs to the field of disease therapy, and more specifically to the field of cancer therapy. In particular, the present invention belongs to a method for determining the sensitivity of a condition, preferably cancer, to a therapeutic agent and/or a method for determining whether a subject suffers from immune ageing and/or immunosenescence.

FIGURE 3

**Description**

**Technical field**

The present invention belongs to the field of disease therapy, and more specifically to the field of cancer therapy. In particular, the present invention belongs to a method for determining the sensitivity of a condition, preferably cancer, to a therapeutic agent and/or a method for determining whether a subject suffers from immune ageing and/or immunosenescence.

**Background of the Invention**

Immune checkpoint inhibitors (ICI), such as anti-PD-1/PD-L1 and anti-CTLA-4, emerged around 2010 as a new standard of care for several cancers. Today, they are prescribed for more than 20 indications, including melanoma, lung, renal, bladder, and head and neck cancers. Certain biomarkers, such as PD-L1 tumor proportion score ≥50%, guide the use anti-PD-1 pembrolizumab as a first-line treatment for metastatic non-small-cell lung cancer (Reck M, Rodriguez-Abreu D, Robinson AG, Hui R, Csőszi T, Fülöp A, et al. Pembrolizumab versus Chemotherapy for PD-L1-Positive Non-Small-Cell Lung Cancer. N Engl J Med. 2016;375:1823-33 [1], Subbiah V, Solit DB, Chan TA, Kurzrock R. The FDA approval of pembrolizumab for adult and pediatric patients with tumor mutational burden (TMB) ≥10: a decision centered on empowering patients and their physicians. Annals of Oncology. 2020;31:1115-8 [2]). The improved response, survival rates, quality of life, and reduced side effects with ICIs, compared to chemotherapy, make them particularly appealing for treating older cancer patients. Older adults are more likely to have severe chemotherapy toxicities than younger ones (Beinse G, Reitter D, Segaux L, Carvahlo-Verlinde M, Rousseau B, Tournigand C, et al. Potential drug-drug interactions and risk of unplanned hospitalization in older patients with cancer: A survey of the prospective ELCAPA (ELderly CAncer PAtients) cohort. Journal of Geriatric Oncology. 2020;11:586-92 [3]). However, treatment decisions in older adults remain more complex than in younger ones, due to the heterogeneity of their health status and lack of evidence-based data in this population (Soubeyran P, Bellera C, Paillaud E. Achieving harmony in oncological geriatric assessment - Should we agree on a best set of tools? Journal of Geriatric Oncology. 2023;14:101482 [4]). As ICIs reached a growing place in the therapeutic arsenal to treat older cancer patients, several studies suggest that the benefits and toxicities in this population are comparable to those in younger patients (Pawelec G. Does patient age influence anti-cancer immunity? Semin Immunopathol. 2019;41:125-31 [5]). Nonetheless, factors influencing this population's response need to be precisely determined.

First, data on the impact of immunological ageing on ICI treatment response and toxicities are lacking. Immune ageing is a complex, multifactorial process characterized by functional and structural alterations in the immune system that occur with ageing. These modifications include a decline in the lymphocytes' proliferative capacity, impaired antigen-presenting cell function, reduced diversity of the lymphocyte repertoire, and alterations in the inflammatory responses (Liu Z, Liang Q, Ren Y, Guo C, Ge X, Wang L, et al. Immunosenescence: molecular mechanisms and diseases. Sig Transduct Target Ther. 2023;8:1-16 [6]).

Ageing induces well-characterized phenotypic changes in T lymphocytes (Gattinoni L, Speiser DE, Lichterfeld M, Bonini C. T memory stem cells in health and disease. Nat Med. 2017;23:18-27 [7]; Koch S, Larbi A, Derhovanessian E, Ozcelik D, Naumova E, Pawelec G. Multiparameter flow cytometric analysis of CD4 and CD8 T cell subsets in young and old people. Immun Ageing. 2008;5:1-12 [8] Romero P, Zippelius A, Kurth I, Pittet MJ, Touvrey C, Iancu EM, et al. Four Functionally Distinct Populations of Human Effector-Memory CD8 + T Lymphocytes. J Immunol. 2007;178:4112-9 [9]). Differentiated T lymphocytes are characterized by the loss of expression of the costimulatory molecules CD27 and CD28 (CD27⁻CD28⁻). CD45RA, a protein tyrosine phosphatase receptor, typically found on naïve T cells (while memory T cells express CD45RO), is re-expressed by the most differentiated T lymphocytes, referred to as effector memory CD45RA⁺ (EMRA) cells. CD8⁺ T cells tend to have a higher proportion of EMRA cells compared to CD4⁺ T cells, and this pool increases with ageing. The percentages of EMRA and CD27⁻CD28⁻ among CD8⁺ T cells increase with age (Callender LA, Carroll EC, Bober EA, Akbar AN, Solito E, Henson SM. Mitochondrial mass governs the extent of human T cell senescence. Aging Cell. 2020; 19:e13067 [10]). The overexpression of receptors usually expressed by NK cells (that we will refer to as NK receptors), such as CD57 and KLRG1, as well as NKG2D, NKG2A, and other members of the KIR/KAR receptor family, accumulates on the differentiated T cells such as EMRA. The threshold for T cell activation via the T cell receptor (TCR) is lower in the elderly (Granier C, Gey A, Roncelin S, Weiss L, Paillaud E, Tartour E. Immunotherapy in older patients with cancer. Biomedical Journal. 2021;44:260-71 [11]; Pereira BI, De Maeyer RPH, Covre LP, Nehar-Belaid D, Lanna A, Ward S, et al. Sestrins induce natural killer function in senescent-like CD8 + T cells. Nature Immunology. 2020;1-11 [12].

Additionally, senescent T lymphocytes may express DAP-12, a protein involved in NK cell signaling through its immunoreceptor tyrosine-based activation motif (ITAM). These findings suggest that senescent T lymphocytes shift from TCR to NK signaling, a process seemingly regulated by stress proteins called sestrins. Sestrin-2 accumulates in T cells with senescence in a complex of MAP kinases p38, Erk and JnK (Pereira BI, De Maeyer RPH, Covre LP, Nehar-Belaid D, Lanna A, Ward S, et al. Sestrins induce natural killer function in senescent-like CD8 + T cells. Nature Immunology. 2020;1-11 [12]; Lanna A, Gomes DCO, Muller-Durovic B, McDonnell T, Escors D, Gilroy DW, et al. A sestrin-dependent Erk/Jnk/p38 MAPK activation complex inhibits immunity during ageing. Nat Immunol. 2017;18:354-63 [13]). Furthermore, as dysfunctional CD8[+] T cells accumulate in the tumour microenvironment, ICIs are known to reinvigorate the CD8[+] T cell proliferation and functions (cytokine secretion and cytotoxic functions), by disrupting CTLA-4/CD80-86 or PD-1/PD-L1 inhibitory signals. CD28 plays a key role in the effective mechanism of action of ICI targeting PD-1 [14]. In this context, since CD8[+] T cell senescence involves loss of CD28 and the accumulation of EMRA cells, which have less proliferative potential and exhibit fewer specific responses, the CD8+ T cell senescence may affect the efficacy of anti-PD-1/anti-PD-L1 therapy.

There is therefore a real need in the state of the art to find a mean or a process to detect CD8+ T cell senescence and/or immune ageing and/or immunosenescence. There is also a real need in the state of the art to find a mean or a process to determine the efficiency or susceptibility of a patient to a therapy, for example an anti-PD-1/anti-PD-L1 therapy.

Secondly, the relationship between immune ageing and chemotherapy, and its clinical relevance, needs further exploration. It has been shown in non-Hodgkin lymphoma (NHL), that an effect of chemotherapy is shorten the telomere length in CD8+ T cells (Lee J-J, Nam C-E, Cho S-H, Park K-S, Chung I-J, Kim H-J. Telomere length shortening in non-Hodgkin's lymphoma patients undergoing chemotherapy. Ann Hematol. 2003;82:492-5 [15]), suggesting that chemotherapy induces CD8+ T cell senescence. Despite the supposed underlying mechanism of cellular senescence induction by chemotherapy is via DNA damage, the relationship between T cell senescence and chemotherapy is not well explored with clinical relevance.

There is therefore a real need in the state of the art to find a mean or a process the sensitivity of a condition, preferably cancer, to a therapeutic agent. There is also a real need in the state of the art to find a mean or a process to determine the sensitivity of a condition, preferably cancer, to a therapeutic agent when the subject to be treated has been previously treated by / or received chemotherapy. There is also a real need in the state of the art to find a mean or a process to detect immune ageing and/or immunosenescence in a subject.

## Detailed description of the invention

The aim of the present invention is precisely to meet these needs by providing a method for determining from a biological sample the sensitivity of a condition, preferably cancer, to a therapeutic agent and also a method for detecting immune ageing and/or immunosenescense in a subject.

The aim of the present invention is also providing a method for detecting immune ageing and/or immunosenescense in a subject.

After intensive research, the applicants have characterized from a biological sample immune ageing and/or immunosenescense. In particular, the applicants have characterized CD8[+] T cell senescence with both EMRA and NK receptors and sestrin-2 in older cancer patients. The inventors have also demonstrated and characterized CD8[+] T cell senescence in older cancer patients before treatment with Immune checkpoint inhibitors (ICI), in particular anti-PD-1/PD-L1, using unsupervised-guided cytometry analysis and demonstrated its association with previous chemotherapy treatment and response to ICI therapy.

Thus, after intensive research, the applicants have developed an inventive approach, allowing to determine, from a biological sample, whether a subject suffers or has immune ageing and/or immunosenescense with the calculation of a particular cells ratio. The applicants have also developed an inventive approach which allows to determine, from a biological sample, whether an anti-cancer chemotherapy induce in a subject immune ageing or immunosenesce. In other words, the applicants have also developed an inventive approach which allows to determine, from a biological sample whether a subject suffers or has ageing or immunosenesce whatever is cause.

Advantageously, the method developed by the applicant does not involve any comparison to a reference value, for example built up from non pathological subject and/or from subject which does not suffer from immune ageing and/or immunosenescense.

The inventors have also surprisingly demonstrated that anticancer drug and/or anti-cancer agent ,preferably chemotherapy, could also induce immunosenescence in a subject.

The present invention advantageously provides a new diagnosis tool for detecting immune ageing and/or immunosenescense.

The applicants have also surprisingly demonstrated that the value of a particular cells ratio advantageously allows determining whether a subject suffering from a disease, in particular a cancer, will be responsive to an immunotherapy. In other words, the applicants have surprisingly demonstrated that the value of a particular cells ratio allows determining the sensitivity of a condition or disease, preferably cancer, to a therapeutic agent, in particular an immunotherapy. Advantageously, the present invention significantly enhances clinical decision-making, adapt the treatment of the condition and improve patient outcomes.

An object of the invention is a method for determining from a biological sample the sensitivity of a condition, preferably cancer, to a therapeutic agent.

In particular, an object of the invention is a method, preferably an in-vitro or ex-vivo method, for determining from a biological sample the sensitivity of a condition, preferably cancer, to a therapeutic agent comprising:

a) determining in a biological sample the amount of CD57$^+$ EMRA CD8$^+$ T cells and the amount of cells CD8$^+$ T cells,
b) calculating the percentage P1 of CD57$^+$ EMRA CD8$^+$ T cells / CD8$^+$ T cells in said biological sample according to the following formula:

$$P1= (CD57^+ \text{ EMRA CD8}^+ \text{ T cells} / \text{CD8}^+ \text{ T cells})*100.$$

The inventors have demonstrated that when percentage P1 is under 10% the condition, preferably cancer, is sensitive to said therapeutic agent. In other words, the inventors have demonstrated that when result or value of P1 is less than 10% the condition, preferably cancer, is sensitive to a therapeutic agent.

An object of the invention is a method, preferably an in-vitro or ex-vivo method, for determining, from a biological sample, the sensitivity of a condition, preferably cancer, to a therapeutic agent comprising:

a) determining in a biological sample the amount of CD57$^+$ EMRA CD8$^+$ T cells and the amount of cells CD8$^+$ T cells
b) calculating the percentage P1 of CD57$^+$ EMRA CD8$^+$ T cells / CD8$^+$ T cells in said biological sample according to the following formula

$$P1= (CD57^+ \text{ EMRA CD8}^+ \text{ T cells} / \text{CD8}^+ \text{ T cells})*100$$

wherein when the percentage P1 is under 10% , the condition, preferably cancer, is sensitive to said therapeutic agent. In other words, the condition, preferably cancer, is sensitive to said therapeutic agent when the percentage P1 is below 10%.

Advantageously, the inventors demonstrated that the method of the invention allows to determine the sensitivity of a condition, preferably cancer, to a therapeutic agent with better sensitivity, specificity, positive predictive value and a negative predictive value than the known method.

Advantageously, the inventors demonstrated that when the value of P1 more than 10%, the condition, preferably cancer, is not responsive or sensitive to a therapeutic agent. In particular, the inventors have demonstrated that when the value of P1 more than 10%, the therapeutic agent is not efficient and the condition is not sensitive to the therapeutic agent with a sensitivity over 61% a specificity over 45%, a positive predictive value over 64%, preferably over 64.7% and a negative predictive value over 41%, preferably over 41.7%.

Advantageously, the inventors characterize immune ageing and/or immunosenescent, and CD8+ T cell senescence in older cancer patients, in particular when treated with anti-PD-1/PD-L1.

Advantageously, by accurately detecting and determining the ratio between CD57$^+$ EMRA CD8$^+$ T cells / CD8$^+$ T cells in a biological sample, the inventors demonstrated surprisingly that this ratio allows determining whether a subject suffers from immune ageing and/or immunosenescense. Advantageously, by accurately determining the ratio between CD57$^+$ EMRA CD8$^+$ T cells / CD8$^+$ T cells, this ratio significantly enhances clinical decision-making and improves patient outcomes.

An object of the invention is thus a method for detecting immune ageing and/or immunosenescense in a subject.

In particular, an object of the invention is a method, preferably an in-vitro or ex-vivo method, for detecting immune ageing and/or immunosenescense in a subject comprising:

i. searching or determining or quantifying in a biological sample the amount of CD57$^+$ EMRA CD8$^+$ T cells and the amount of cells CD8$^+$ T cells
ii. calculating the percentage P3 of CD57$^+$ EMRA CD8$^+$ T cells / CD8$^+$ T cells in said biological sample according to the following formula

$$P3= (CD57^+ \text{ EMRA CD8}^+ \text{ T cells} / \text{CD8}^+ \text{ T cells})*100$$

The inventors have advantageously demonstrated that when the percentage P3 is over 10%, the subject has or suffers from immune ageing and/or immunosenescense.

An object of the invention is a method, preferably an in-vitro or ex-vivo method, for detecting immune ageing and/or immunosenescense in a subject comprising:

i. searching or determining or quantifying in a biological sample the amount of CD57$^+$ EMRA CD8$^+$ T cells and the amount of cells CD8$^+$ T cells

ii. calculating the percentage P3 of CD57$^+$ EMRA CD8$^+$ T cells / CD8$^+$ T cells in said biological sample according to the following formula

$$P3= (CD57^+ EMRA CD8^+ \text{ T cells} / CD8^+ \text{ T cells})*100$$

when the percentage P3 is over 10% the subject has or suffers from immune ageing and/or immunosenescense.

**Definitions**

To facilitate an understanding of the present invention, a number of terms and phrases are defined below:
As used herein other than the claims, the terms "a," "an," "the," and/or "said" means one or more. As used herein in the claim(s), when used in conjunction with the words "comprise," "comprises" and/or "comprising," the words "a," "an," "the," and/or "said" may mean one or more than one. As used herein and in the claims, the terms "having," "has," "is," "have," "including," "includes," and/or "include" has the same meaning as "comprising," "comprises," and "comprise." As used herein and in the claims "another" may mean at least a second or more. As used herein and in the claims, "about" refers to any inherent measurement error or a rounding of digits for a value (e.g., a measured value, calculated value such as a ratio), and thus the term "about" may be used with any value and/or range.

The phrase "a combination thereof", "a mixture thereof" and such like following a listing, the use of "and/or" as part of a listing, a listing in a table, the use of "etc." as part of a listing, the phrase "such as," and/or a listing within brackets with "e.g.," or i.e., refers to any combination (e.g., any sub-set) of a set of listed components, and combinations and/or mixtures of related species and/or embodiments described herein though not directly placed in such a listing are also contemplated. Such related and/or like genera(s), sub-genera(s), specie(s), and/or embodiment(s) described herein are contemplated both in the form of an individual component that may be claimed, as well as a mixture and/or a combination that may be described in the claims as "at least one selected from," "a mixture thereof" and/or "a combination thereof."

As used herein, the term "and/or" means any one of the items, any combination of the items, or all of the items with which this term is associated.

As used herein, the term "about" refers to a variation of ±5-10% of the value specified. For example, "about 50" percent can in some embodiments carry a variation from 45 to 55 percent. For integer ranges, the term "about" can include one or two integers greater than and/or less than a recited integer. Unless indicated otherwise herein, the term "about" is intended to include values, e.g., weight percents, proximate to the recited range that are equivalent in terms of the functionality of the individual ingredient, the composition, or the embodiment.

As will be understood by the skilled artisan, all numbers, including those expressing quantities of ingredients, properties such as molecular weight, reaction conditions, and so forth, are approximations and are understood as being optionally modified in all instances by the term "about." These values can vary depending upon the desired properties sought to be obtained by those skilled in the art utilizing the teachings of the descriptions herein. It is also understood that such values inherently contain variability necessarily resulting from the standard deviations found in their respective testing measurements.

As will be understood by one skilled in the art, for any and all purposes, particularly in terms of providing a written description, all ranges recited herein also encompass any and all possible subranges and combinations of subranges thereof, as well as the individual values making up the range, particularly integer values. A recited range (e.g., weight percents) includes each specific value, integer, decimal, or identity within the range. Any listed range can be easily recognized as sufficiently describing and enabling the same range being broken down into at least equal halves, thirds, quarters, fifths, or tenths. As a non-limiting example, each range discussed herein can be readily broken down into a lower third, middle third and upper third, etc.

As will also be understood by one skilled in the art, all language such as "up to," "at least," "greater than," "less than," "more than," "or more," and the like, include the number recited and such terms refer to ranges that can be subsequently broken down into subranges as discussed above. In the same manner, all ratios recited herein also include all sub-ratios falling within the broader ratio

An "effective amount" refers to an amount effective to treat a disease, disorder, and/or condition, or to bring about a recited effect. For example, an amount effective can be an amount effective to reduce the progression or severity of the condition or symptoms being treated. Determination of a therapeutically effective amount is well within the capacity of persons skilled in the art. The term "effective amount" is intended to include an amount of a compound described herein, or an amount of a combination of compounds described herein, e.g., that is effective to treat or prevent a disease or disorder, or to treat the symptoms of the disease or disorder, in a host. Thus, an "effective amount" generally means an amount that provides the desired effect.

The terms "treating", "treat" and "treatment" include (i) preventing a disease, pathologic or medical condition from occurring (e.g., prophylaxis); (ii) inhibiting the disease, pathologic or medical condition or arresting its development; (iii) relieving the disease, pathologic or medical condition; and/or (iv) diminishing symptoms associated with the disease, pathologic or medical condition. Thus, the terms "treat", "treatment", and "treating" extend to prophylaxis and include prevent, prevention, preventing, lowering, stopping or reversing the progression or severity of the condition or symptoms being treated. As such, the term "treatment" includes medical, therapeutic, and/or prophylactic administration, as appropriate.

A "sample" isolated from a subject may be a biological sample (e.g., a cell, a tissue, an organ, a body fluid (e.g., blood, lymphatic fluid, etc.), a digestive fluid, sputum, an alveoli/bronchial washing fluid, urine, or stool), biopsy, tissue biopsy and tissue section may be a sample containing cancer cells, or may be a sample containing no cancer cells.

"Biological sample" means any biological liquid, for example, it may be a sample of blood, for example peripheral blood, including whole blood or fractioned blood, plasma, serum, ascites, a spleen biopsy, tissue biopsy, tissue section, for example colon tissue section or a tumor sample. Preferably, it can be peripheral blood. The biological sample may be blood comprising PBMC peripheral mononuclear cells. For example, when the biological sample is blood comprising PBMC peripheral mononuclear cell, the number of the PBMC peripheral mononuclear cells in the biological sample can be at least 100 000, for example at least 500 000 PBMC peripheral mononuclear cells. The biological sample may be a biological sample previously taken from a patient or subject. According to the invention, when the biological sample is a blood sample, it may be a biological sample previously taken from a vein on a patient or subject.

According to the invention, the biological sample may be a sample taken from 1 minute to 48 hours, for example from 1 minute to 24 hours, for example from 1 hour to 6 hours prior to its use in the method according to the invention. According to the invention the volume of the biological sample can be from $10\mu l$ to 10 ml, for example from $12\mu l$ to 5 ml, for example from $100\mu l$ to 2 ml. The volume of the biological sample can be at least $300\mu l$, for example from $300\mu l$ to 2ml, for example from 300 to $500\mu l$. For example, when the biological sample is blood, the volume of the biological sample can be at least $100\mu l$, for example at least $200\mu l$.

According to the invention, the biological sample may be frozen and/or thawed prior to its use in the method according to the invention. When the biological sample is frozen prior to its use in the method according to the invention, it may be used 1 hour to 6 hours after thawing in the method according to the invention. When the biological sample is frozen prior to its use in the method according to the invention, it may be used in the method according to the invention at least 72 hours, for example 1 week, for example 1 month, for example 1 year, for example 10 years, for example 20 years after freezing.

As used herein, "patient" or "subject" may be an animal, mammal, or human, preferably a human.

A "subject" or a "patient" may be an animal, mammal, or human, preferably a human suffering or who has a possibility of suffering from a condition.

As used herein, "patient" or "subject" refers to any individual likely to have, for example, a condition, for example a cancer. It may be also a human who has a possibility of having the cancer recur or a human who has a possibility of suffering from the cancer., e.g. a human of any sex and/or age. For example, it could be a human, such as an infant, a baby, a child, a teenager, an adult. For example, it may be a human aged between 7 days and 99 years. It can be, for example, a child with an age ranging from 7 days to 12 years, an adolescent with an age ranging from 12 to 17 years, an adult with an age over 17 years, for example over 25 years, for example over 35 years, for example over 45 years, for example over 50 years, for example over 65 years, for example over 70 years. Preferably, the "patient" or "subject" is an adult over 50 years.

In other words, a "subject" or a "patient" suffering or who has a possibility of suffering from a condition, preferably a cancer, to be assessed to determine whether said condition, preferably a cancer, is sensitive to a therapeutic agent is usually a human (a cancer patient) but may be a human who has a possibility of having the cancer recur or a human who has a possibility of suffering from the cancer. Preferably a patient means a patient having a cancer, for example a human or an animal, regardless of age and sex. For example, it may be a human aged between 7 days and 99 years. It can be, for example, a child with an age ranging from 7 days to 12 years, an adolescent with an age ranging from 12 to 17 years, an adult with an age over 17 years, for example over 25 years, for example over 35 years, for example over 45 years, for example over 55 years, for example over 65 years, for example over 70 years, for example over 79 years. Preferably, the "patient" or "subject" is an adult over 50 years

The subject may, prior to the determination from a biological sample immune ageing and/or immunosenescense, have received at least one line of anti-cancer therapy. The subject may, prior to the determination from a biological sample the sensitivity of a condition, preferably cancer, to a therapeutic agent, may have received at least one line of anti-cancer therapy. For example, the subject may previously receive at least one line of anti-cancer therapy for the treatment of advanced and/or metastatic cancer. For example, the subject may have previously received treatment for cancer with anticancer drug and/or anti-cancer agent. It may be for example 5 fluoro-uracile (5FU), oxaliplatine. It may be a cytotoxic drug. As used herein, the term "cytotoxic drug" refers to a molecule that when entering in contact with a cell, optionally upon internalization into the cell, alters a cell function, for example cell growth and/or proliferation and/or differentiation and/or metabolism , for example protein and/or DNA synthesis, in a detrimental way or leads to cell death. As used herein, the term "cytotoxic drug" encompasses toxins, in particular cytotoxins. It may be, for example, a compound selected from the group comprising calicheamycin, dolastin 10, dolastin 15, auristatin E, auristatin EB (AEB), auristatin EFP (AEFP), monomethyl auristatin F (MMAF), monomethylauristatin-D (MMAD), monomethyl auristatin E (MMAE), and 5-benzoylvaleric acid-AE ester (AEVB) and duocarmycin; nitrogen mustard analogues for example cyclophosphamide, melphalan, ifosfamide or trofosfamide; ethylenimines such as thiotepa; nitrosoureas for example carmustine; alkylating agents for example temozolomide or dacarbazine; folate-like metabolic antagonists such as methotrexate or raltitrexed; purine analogues for example thioguanine, cladribine or fludarabine; pyrimidine analogues for example fluorouracil, tegafur or gemcitabine; vinca alkaloids for example vinblastine, vincristine or vinorelbine and analogues thereof; podophyllotoxin derivatives for example etoposide, taxans, docetaxel or paclitaxel; anthracyclines for example doxorubicin, epirubicin, idarubicin and mitoxantrone, and analogues thereof; other cytotoxic antibiotics for example bleomycin and mitomycin; platinum compounds for example cisplatin, carboplatin and oxaliplatin; pentostatin, miltefosine, estramustine, topotecan, irinotecan and bicalutamide, and toxins for example ricin toxin, liatoxin and Vero toxin; or any combination thereof.

In the present, condition or disease may be used interchangeably. It may be for example a condition or disease selected from the group comprising cancer, infection, organ transplantation, bone marrow transplantation, auto-immune disease, long term treatment with immunosuppressive drug, chronic viral disease for example human immunodeficiency virus (HIV), hepatitis C virus (HCV), preferably cancer.

It may be any condition or disease known from one skilled in the art that could affect the immunity of a subject or a patient. It may be for example any condition known from one skilled in the art that could affect the immune ageing and/or immunosenescense. It may be for example any condition known from one skilled in the art that could induce the immune ageing and/or immunosenescense. For example, It may be a condition selected from the group comprising infection, organ transplantation, bone marrow transplantation, long term treatment with immunosuppressive drug, chronic viral disease for example human immunodeficiency virus (HIV), hepatitis C virus (HCV).

It may be for example any condition known from one skilled in the art that could be induced by immune ageing and/or immunosenescense. It may be for example a condition or disease selected from the group comprising cancer, infection, chronic viral disease for example human immunodeficiency virus (HIV), hepatitis C virus (HCV), preferably cancer.

In the present "cancer" may be any cancer of any organ known from one skilled in the art. It may be for example solid cancer. It may be for example any disease involving abnormal cell growth with the potential to invade or spread to other parts of the body. It may be for example any cancer of any organ known from one skilled in the art. It may be for example cancer of any organ or tissue of a human or of an animal. It may be for example a cancer comprising at least one solid tumor and/or a cancer involving hemopathy. It may be for example a cancer of any organ selected from the group comprising mouth, liver, esophagus, Ear Nose and Throat (ENT), head, neck, trachea, stomach, kidney, colon, colorectal, pancreas, mesothelioma, cervical, uterine, gall bladder, bladder, prostate, rectum, lung, renal, skin, breast, sarcoma, and lymphomas. It may be for example a cancer of any organ selected from the group comprising mouth, esophagus, trachea, pancreas, stomach, gall bladder, small intestine, colon, rectum, liver, lung, skin, breast. The "cancer" may be a solid tumor or a humoral tumor. Examples of the solid tumors include lung cancer (non-small cell lung cancer, small cell lung cancer, pulmonary adenocarcinoma, small bronchopulmonary lung cell carcinoma, mesothelioma, etc.), thyroid cancer (medullary thyroid cancer, papillary thyroid cancer, etc.), colon cancer, central nerve cancer, brain cancer, for example glioblastoma, and skin, for example melanoma, humoral tumors include, for example, blood cancer and leukemia. The state may be progressive, metastatic, or recurrent. Preferably, it may be a cancer of an organ selected from the group comprising renal, skin, colon, stomach, bladder, kidney, lung, ear nose and throat and mesothelium.

In the present, Infection that could induce the immune ageing and/or immunosenescense may be for example selected from the group comprising a bacterial infection, a viral infection and a parasitic infection. It may be for example an infection selected from the group comprising Human Immunodeficiense Virus (HIV) or AIDS, Severe Acute Respiratory Syndrome-CoV virus or COVID-19, tuberculosis and leismaniasis.

In the present, organ transplantation that could induce the immune ageing and/or immunosenescense may be an organ transplantation selected from the group comprising renal, hepatic and heart transplantation.

Immunosuppressive drug that could induce immunosenescense may be, for example, selected from the group comprising methotrexate, azathioprine, mercaptopurine, dactinomycin, ciclosporin and zotarolimus.

In the present, T cell means T lymphocyte. It is a type of white blood cell. T cells are part of the immune system and develop from stem cells that are in the bone marrow. They are part of the specific immune response and protect the body from infection and cancer formation. T cells are characterized by the cell surface expression of the cluster of differentiation 3 (CD3). CD3 is a component of the TCR complex. Surface membrane CD3 is a pan-T-cell marker. For example, T cell may be as disclosed in BONIFACINO, Juan S., CHEN, Catherine, LIPPINCOTT-SCHWARTZ, Jennifer, et al. Subunit interactions within the T-cell antigen receptor: clues from the study of partial complexes. Proceedings of the National Academy of Sciences, 1988, vol. 85, no 18, p. 6929-6933 [30].

In the present CD8, also known as cytotoxic T lymphocyte, means Cluster of Differentiation 8 which is a glycoprotein comprising of two subunits, CD8A (alpha chain) and CD8B (beta chain), expressed on the surface of T cells, and is encoded by the CD8A and CD8B genes. For example, CD8 is as disclosed in Sun J, Kavathas PB. Comparison of the roles of CD8 alpha alpha and CD8 alpha beta in interaction with MHC class I. J Immunol. 1997 Dec 15;159(12):6077-82. PMID: 9550407 [31]. CD8+ T cells can be characterized by CD8 expression, optionally by CD8 and CD3 expression.

In the present CD8+T cell, also designed "cytotoxic T cells", means cell of the immune system carrying on its surface Cluster of Differentiation 8 (CD8) for example CD8+T cell is as disclosed in Raskov, H., Orhan, A., Christensen, J.P. et al. Cytotoxic CD8+ T cells in cancer and cancer immunotherapy. Br J Cancer 124, 359-367 (2021). https://doi.org/10.1038/s41416-020-01048-4 [32].

In the present CD57 means Cluster of Differentiation 57 (CD57) which belongs to the glycoprotein family and is usually expressed on the cell surface of Natural Killer (NK) cells. The CD57 antigen, also called HNK-1, LEU-7 or L2, is a 100-115 kD terminally sulfated carbohydrate epitope that was originally reported as a marker of human natural killer cells CD57 can also be found in CD8 T cells. Like carbonhydrates, it represents a binding site and had cellular activities like recognition and adhesion to other cell or microorganisms or toxin, for example. For example, Cluster of Differentiation 57 is as disclosed in Kared H, Martelli S, Ng TP, Pender SL, Larbi A. CD57 in human natural killer cells and T-lymphocytes. Cancer Immunol Immunother. 2016 Apr;65(4):441-52. doi: 10.1007/s00262-016-1803-z. Epub 2016 Feb 5. PMID: 26850637; PMCID: PMC11029668 [33] and/or Kared, H., Martelli, S., Ng, T.P. et al. CD57 in human natural killer cells and T-lymphocytes. Cancer Immunol Immunother 65, 441-452 (2016). https://doi-org.proxy.insermbiblio.inist.fr/10.1007/s00262-016-1803-z) [34]

In the present "EMRA cell" means Effector Memory CD45RA+ T cell. It is a T cell expressing on its surface Cluster of Differentiation 45RA (CD45RA) and not expressing Cluster of Differentiation 27 (CD27) and Cluster of Differentiation 28 (CD28), and optionally not expressing Cluster of Differentiation 62L (CD62L) and Chemokine (C-C Motif) Receptor 7 (CCR7). For example, EMRA cell may be as disclosed in Koch, S., Larbi, A., Derhovanessian, E. et al. Multiparameter flow cytometric analysis of CD4 and CD8 T cell subsets in young and old people. Immun Ageing 5, 6 (2008). https://doi.org/10.1186/1742-4933-5-6 [35] and/or Romero P, Zippelius A, Kurth I, Pittet MJ, Touvrey C, Iancu EM, et al. Four Functionally Distinct Populations of Human Effector-Memory CD8 + T Lymphocytes. J Immunol. 2007;178:4112-9 [36].

In the present Cluster of Differentiation 27 (CD27) is a costimulatory molecule expressed at cell surface. Cluster of Differentiation 27 (CD27) may be as disclosed in HINTZEN, Rogier Q., LENS, Susanne M., BECKMANN, M. Patricia, et al. Characterization of the human CD27 ligand, a novel member of the TNF gene family. Journal of immunology (Baltimore, Md.: 1950), 1994, vol. 152, no 4, p. 1762-1773 [37].

In the present Cluster of Differentiation 28 (CD28) is a costimulatory molecule expressed on cell surface. Cluster of Differentiation 28 (CD28) may be as disclosed in Esensten JH, Helou YA, Chopra G, Weiss A, Bluestone JA. CD28 Costimulation: From Mechanism to Therapy. Immunity. 2016 May 17;44(5):973-88. doi: 10.1016/j.immuni.2016.04.020. PMID: 27192564; PMCID: PMC4932896 [38].

In the present Cluster of Differentiation 62L (CD62L) also mentioned as L-selectin is a type-I transmembrane glycoprotein and cell adhesion molecule that is expressed on the surface on most circulating leukocytes. Cluster of Differentiation 62L (CD62L) may be as disclosed in Ivetic A, Hoskins Green HL, Hart SJ. L-selectin: A Major Regulator of Leukocyte Adhesion, Migration and Signaling. Front Immunol. 2019 May 14;10:1068. doi: 10.3389/fimmu.2019.01068. PMID: 31139190; PMCID: PMC6527602 [39].

In the present Chemokine (C-C Motif) Receptor 7 (CCR7) is a chemokine receptor expressed at the cell surface. Chemokine (C-C Motif) Receptor 7 (CCR7) may be as disclosed in Campbell JJ, Murphy KE, Kunkel EJ, Brightling CE, Soler D, Shen Z, Boisvert J, Greenberg HB, Vierra MA, Goodman SB, Genovese MC, Wardlaw AJ, Butcher EC, Wu L. CCR7 expression and memory T cell diversity in humans. J Immunol. 2001 Jan 15;166(2):877-84. doi: 10.4049/jimmunol.166.2.877. PMID: 11145663 [40].

In the present CD57+ EMRA CD8+ T cell means EMRA CD8+ T cells expressing on its surface Cluster of differentiation CD57. For example, CD57+ EMRA CD8+ T cell is as disclosed in Verma K, Ogonek J, Varanasi PR, Luther S, Bünting I, Thomay K, Behrens YL, Mischak-Weissinger E, Hambach L. Human CD8+ CD57- TEMRA cells: Too young to be called "old". PLoS One. 2017 May 8;12(5):e0177405. doi: 10.1371/journal.pone.0177405. PMID: 28481945; PMCID: PMC5421808 [41].

In the present, methods for detecting and/or quantifying protein biomarkers and/or cells according to the invention, include, but are not limited to, immuno-analysis immunofluorescence, immunoglobulin-mediated assays, Mass cytometry Imaging and other techniques known in the art. It can be known method for detecting and/or quantifying cells, for example flow cytometry, mass cytometry, spectral cytometry, microscopy, any automated imaging system, or any combination thereof. One skilled in the art, taking into consideration his technical knowledge, knows method for detecting and/or quantifying cells, for example T cells, for example CD8+ T cells, CD57+ EMRA CD8+ T cell and/or CD57+ GRZB+ EMRA CD8+ T cell in a biological sample. For example, it may be a method by immunofluorescence comprising a step of labelling cells with antibodies, for example flow cytometry antibodies, for example as disclosed in Mousset CM, Hobo W, Woestenenk R, Preijers F, Dolstra H, van der Waart AB. Comprehensive Phenotyping of T Cells Using Flow Cytometry. Cytometry A. 2019 Jun;95(6):647-654. doi: 10.1002/cyto.a.23724. Epub 2019 Feb 4. PMID: 30714682 [42]; and/or Zacharias ZR, Houtman JCD. OMIP-099: 31-color spectral flow cytometry panel to investigate the steady-state phenotype of human T cells. Cytometry A. 2024 Jan;105(1):10-15. doi: 10.1002/cyto.a.24799. Epub 2023 Oct 9. PMID: 37814476; PMCID: PMC10842108 [43].

The presence and/or detection of CD8+ T cells in a biological sample may be determined using detecting agents which can be for example antibodies, preferably, anti-CD8 antibodies. It may be any anti-CD8 antibodies known from one skilled in the art and/or commercially available. It may be for example anti-CD8 antibody commercialized by BD biosciences under the reference RPA-T8, clone 563823, the anti-CD8 antibody commercialized by Invitrogen under the reference CD8a Monoclonal Antibody (RPA-T8), PE, eBioscience 12-0088-42.

The presence and/or detection of CD8+ T cells in a biological sample may be determined using detecting agents which can be for example antibodies, preferably, anti-CD8 antibodies, optionally with anti-T(CD3+) antibodies. Anti-T(CD3+) antibodies may be any anti-CD3 antibodies known from one skilled in the art and or commercially available, it may be for example anti-CD3 antibody commercialized by BD biosciences under the reference BD Horizon™ BUV395 Mouse Anti-Human CD3 clone UCHT1, anti-CD3 antibody commercialized by Merck under the reference C7930.

The presence and/or detection of CD57+ EMRA CD8+ T cells in a biological sample may be determined using detecting agents selected from the group comprising antibodies, preferably, anti-CD57 antibodies, anti-CD45RA antibodies, anti-CD62L and anti-CD28 and anti-CD27 antibodies, or a combination thereof. It may be any anti-CD57 antibodies known from one skilled in the art and/or commercially available, it may be for example anti-CD57 antibody commercialized by BD biosciences under the reference BD Horizon™ PE-CF594 Mouse Anti-Human CD57 Clone NK-1 562488, an anti-CD57 antibody commercialized by Invitrogen under the reference CD57 Monoclonal Antibody (TB01 (TBO1)), PE, eBioscience™ 12-0577-42. It may be any anti-CD45RA antibodies known from one skilled in the art and/or commercially available, it may be an anti-CD45RA antibody commercialized by Biolegend under the reference Brilliant Violet 421™ anti-human CD45RA Antibody, an anti-CD45RA antibody commercialized by Invitrogen under the reference CD45RA Monoclonal Antibody (HI100), eBioscience™ 12-0458-42. It may be any anti-CD62L antibodies known from one skilled in the art and/or commercially available, it may be an anti-CD62L antibody commercialized by Invitrogen under the reference CD62L (L-Selectin) Monoclonal Antibody (MEL-14), Functional Grade, eBioscience™ 16-0621-86, an anti-CD62L antibody commercialized by Sigma-Aldrich under the reference Anti-CD62L (L-Selectin) Antibody (mouse), APC, clone MEL-14, MABF715. It may be any anti-CCR7 antibodies known from one skilled in the art and/or commercially available, it may be an anti-CCR7 antibody commercialized by Invitrogen under the reference CD197 (CCR7) Monoclonal Antibody (4B12), PE, eBioscience™ 12-1971-82, an anti-CCR7 antibody commercialized by Invitrogen under the reference CD197 (CCR7) Monoclonal Antibody (3D12), PE, eBioscience™, 12-1979-42. It may be any anti-CD28 antibodies known from one skilled in the art and/or commercially available, it may be an anti-CD28 antibody commercialized by BD biosciences under the reference BD Pharmingen™ PE-Cy™5 Mouse Anti-Human CD28 Clone CD28.2, 555730, an anti-CD28 antibody commercialized by Invitrogen under the reference CD28 Monoclonal Antibody (CD28.2), eBioscience™, 14-0289-82. It may be any anti-CD27 antibodies known from one skilled in the art and/or commercially available, it may be an anti-CD27 antibody commercialized by BD Biosciences under the reference BD™ PE Mouse Anti-Human CD27 Clone L128, 340425.

According to the invention, the method for determining from a biological sample the sensitivity of a condition, preferably cancer, to a therapeutic agent can further comprises:

c) determining in a biological sample the amount of CD57+ GRZB+ EMRA CD8+ T cells and

d) calculating the percentage P2 of CD57+ GRZB+ EMRA CD8+ T cells / CD8+ T cells in said biological sample according to the following formula

$$P2 = (CD57+ \ GRZB+ \ EMRA \ CD8+ \ T \ cells \ / \ CD8^+ \ T \ cells)*100.$$

According to the invention, the biological sample is a biological sample as defined above.

Advantageously, the inventors demonstrated when the percentage P2 is less than 10 percent, the inventors demonstrated that the method of the invention allows to determine the sensitivity of a condition, preferably cancer, to a therapeutic agent. In particular, the inventors have demonstrated that when the percentage P2 is less than 10 percent, the condition is sensitive to a therapeutic agent.

Advantageously, the inventors demonstrated when the percentage P2 is more than 10 the condition, preferably cancer, is not responsive or sensitive to a therapeutic agent. In particular, the inventors have demonstrated that when the value of P1 more than 10%, the therapeutic agent is not efficient and the condition is not sensitive to the therapeutic agent percent, the inventors demonstrated that the method of the invention allows to determine the sensitivity of a condition, preferably cancer, to a therapeutic agent with a specificity over 80%, preferably of 81.8%. a positive predictive value over 80%, a negative predictive value over 47%, preferably over 47.4% and a sensibility over 44%, preferably over 44.4%

In the present Granzyme means a family of serine proteases released by immune cells to induce cytolysis and apoptosis in target cells, such as virus-infected cells or tumor cells. Granzyme B (GZMB or GRZB) is a member of the granzyme family and corresponds to a protein having a molecular weight of around 30 kDa encoded by the GZMB gene. For example, granzyme B cell is as disclosed in Cullen, S., Brunet, M. & Martin, S. Granzymes in cancer and immunity. Cell Death Differ 17, 616-623 (2010). https://doi.org/10.1038/cdd.2009.206 [44].

In the present CD57$^+$ GRZB$^+$ EMRA CD8$^+$ T cell means EMRA CD8$^+$ T cell expressing on its surface Cluster of differentiation CD57 and intracellular granzyme-B (GRZB) gene.

According to the invention, the method for detecting immune ageing and/or immunosenescense in a subject can further comprises:

iii. searching and/or determining in the biological sample the amount of CD57$^+$ GRZB$^+$ EMRA CD8$^+$ T cells and

iv. calculating the percentage P4 of CD57$^+$ GRZB$^+$ EMRA CD8$^+$ T cells / CD8$^+$ T cells in said biological sample according to the following formula

$$P4= (CD57^+ \ GRZB^+ \ EMRA \ CD8^+ \ T \ cells \ / \ CD8^+ \ T \ cells)*100.$$

According to the invention, the biological sample is a biological sample as defined above.

According to the invention, the subject is as defined above.

According to the invention, the biological sample used to determine the amount of CD57$^+$ GRZB$^+$ EMRA CD8$^+$ T cells may be identical to or different to the sample used to determine the amount of CD57$^+$ EMRA CD8$^+$ T cells and the amount of cells CD8$^+$ T cells.

Advantageously, the inventors demonstrated when the percentage P4 is more than 10 percent, the inventors demonstrated that the method of the invention allows to determine whether a subject has or suffers from immune ageing and/or immunosenescense.

The presence and/or detection of CD57$^+$ GRZB$^+$ EMRA CD8$^+$ T cells in the biological sample may be determined using detecting agents which can be for example antibodies selected from the group comprising, anti-CD57 antibodies, anti-CD45RA antibodies, anti-CD62L antibodies, anti-CCR7 antibodies, anti-CD27 antibodies, anti-CD28 antibodies, preferably anti-CD57 antibodies, anti-CD45RA antibodies, anti-CD27 antibodies, anti-CD28 antibodies or any combination thereof. It may be any anti-CD57 antibodies known from one skilled in the art and/or commercially available. It may be any anti-CD45RA antibodies known from one skilled in the art and/or commercially available. It may be any anti-CD62L antibodies known from one skilled in the art and/or commercially available. It may be any anti-CCR7 antibodies known from one skilled in the art and/or commercially available. It may be any anti-CD28 antibodies known from one skilled in the art and/or commercially available. It may be any anti-CD27 antibodies known from one skilled in the art and or commercially available. It may be any anti-Granzyme B antibodies known from one skilled in the art and or commercially available.

The presence or absence of each of the cell markers may be denoted by a + or a - sign, respectively

In the present the therapeutic agent may be any compound whatever its form. It may be for example a chemical compound, a peptide, a polynucleic acid, an antibody.

In the present, the therapeutic agent may be anticancer drug and/or anti-cancer agent. It may be for example 5 fluoro-uracile (5FU), oxaliplatine. It may be a cytotoxic drug. As used herein, the term "cytotoxic drug" refers to a molecule that when entering in contact with a cell, optionally upon internalization into the cell, alters a cell function (for example cell growth and/or proliferation and/or differentiation and/or metabolism such as protein and/or DNA synthesis) in a detrimental way or leads to cell death. As used herein, the term "cytotoxic drug" encompasses toxins, in particular cytotoxins. It may be, for example, a compound selected from the group comprising calicheamycin, dolastin 10, dolastin 15, auristatin E, auristatin EB (AEB), auristatin EFP (AEFP), monomethyl auristatin F (MMAF), monomethylauristatin-D (MMAD), monomethyl auristatin E (MMAE), and 5-benzoylvaleric acid-AE ester (AEVB) and duocarmycin; nitrogen mustard analogues for example cyclophosphamide, melphalan, ifosfamide or trofosfamide; ethylenimines such as thiotepa; nitrosoureas for example carmustine; alkylating agents for example temozolomide or dacarbazine; folate-like metabolic antagonists such as methotrexate or raltitrexed; purine analogues for example thioguanine, cladribine or fludarabine; pyrimidine analogues for example fluorouracil, tegafur or gemcitabine; vinca alkaloids for example vinblastine, vincristine or vinorelbine and analogues thereof; podophyllotoxin derivatives for example etoposide, taxans, docetaxel or paclitaxel; anthracyclines for example doxorubicin, epirubicin, idarubicin and mitoxantrone, and analogues thereof; other cytotoxic antibiotics for example bleomycin and mitomycin; platinum compounds for example cisplatin, carboplatin and oxaliplatin; pentostatin, miltefosine, estramustine, topotecan, irinotecan and bicalutamide, and toxins for example ricin toxin, liatoxin and Vero toxin; or any combination thereof.

In the present, the therapeutic agent may be an immunotherapy. It may be for example any immunotherapy known from one skilled in the art. It may be for example an immunotherapy selected from the group comprising pembrolizumab, nivolumab, pidilizumab, avelumab, atezolizumab, or any combination thereof. It may be a PD-1 or PD-L1 antagonist. It may be an anti-PD-1 antibody, for example selected from the group comprising Nivolumab, Pembrolizumab, Cemiplimab, Toripalimab, Sintilimab, Tislelizumab, Envafolimab, Prolgolimab, Serplulimab, Dostarlimab, Camrelizumab and Retifanlimab, or any combination thereof. It may be an anti-PD-L1 antibody, for example selected from the group comprising Atezolizumab, Durvalumab, Avelumab, KN035, Sugemalimab and Cosibelimab, or any combination thereof.

In the present, immune ageing means functional and structural alterations in the immune system that occur with ageing, involving a decrease of the efficiency and functionality of the immune system. One skilled in the art taking into consideration his technical knowledge knows the meaning of immune ageing.

In the present, immunosenscense means functional and structural alterations in the immune system that occur with ageing as disclosed in Liu, Z., Liang, Q., Ren, Y. et al. Immunosenescence: molecular mechanisms and diseases. Sig Transduct Target Ther 8, 200 (2023). https://doi.org/10.1038/s41392-023-01451-2 [6]. One skilled in the art taking into consideration his technical knowledge knows the meaning of immunosenescense.

The immune alteration can be for example a lack of reactivity, for example decrease of cytotoxic degranulation or cytokine secretion, after a specific stimulus and/or a higher threshold of activation of immune cells and/or a lower membrane plasticity and/or a higher reactivity at basal level without specific stimulus that induce inflammageing. For example it may be an alteration of qualitative cellular immune response and an adaptation of the immune response.( Granier, C., Gey, A., Roncelin, S., Weiss, L., Paillaud, E., & Tartour, E. (2021). Immunotherapy in older patients with cancer. biomedical journal, 44(3), 260-271. )

Another object of the invention is an in-vitro or ex-vivo method for determining from a biological sample whether a therapeutic agent induce immunosenscense in a subject comprising:

A. searching and/or determining in a biological sample the amount of $CD57^+$ EMRA $CD8^+$ T cells and/or $CD57^+$ $GRZB^+$ EMRA $CD8^+$ T, and $CD8^+$ T cells before treatment of a subject,

B. searching and/or determining in a biological sample the amount of $CD57^+$ EMRA $CD8^+$ T cells and/or $CD57^+$ $GRZB^+$ EMRA $CD8^+$ T, and $CD8^+$ T cells after treatment of the subject,

C. calculating the percentage P5 of $CD57^+$ EMRA $CD8^+$ T cells / $CD8^+$ T cells in said biological sample before treatment according to the following formula P5=($CD57^+$ $EMRA^+$ $CD8^+$ T cells / $CD8^+$ T cells)*100 and the percentage P6 of $CD57^+$ EMRA $CD8^+$ T cells / $CD8^+$ T cells after treatment according to the following formula P6=($CD57^+$ $EMRA^+$ $CD8^+$ T cells / $CD8^+$ T cells)*100 after treatment and/or

the percentage P7 of $CD57^+$ EMRA$^+$ $GRZB^+$ $CD8^+$ T cells / $CD8^+$ T cells in said biological sample before treatment according to the following formula P7= ($CD57^+$ $GRZB^+$ $EMRA^+$ $CD8^+$ T cells / $CD8^+$ T cells)*100 and the percentage P8 of $CD57^+$ $GRZB^+$ EMRA $CD8^+$ T cells / $CD8^+$ T cells after treatment according to the following formula P8= ($CD57^+$ $GRZB^+$ $CD8^+$ T cells / $CD8^+$ T cells)*100,

D. comparing the value of P5 and P6 according the following formula : P9=P5/P6 and/or the value of P7 and P8 according the following formula P10= P7/P8

wherein when the value of P9 and/or P10 is less than 1, preferably less than 0.6, the therapeutic agent induces immunosenescence.

In the present inducing immunosenscense means altering the immune system of a subject, for example a lack of reactivity, for example decrease of cytotoxic degranulation or cytokine secretion, after a specific stimulus and/or a higher threshold of activation of immune cells and/or a lower membrane plasticity and/or a higher reactivity at basal level without specific stimulus that induce inflammageing. For example, it may be an alteration of the immune system as disclosed in Liu, Z., Liang, Q., Ren, Y. et al. Immunosenescence: molecular mechanisms and diseases. Sig Transduct Target Ther 8, 200 (2023). https://doi.org/10.1038/s41392-023-01451-2 [6] and/or it may be an alteration of qualitative cellular immune response and an adaptation of the immune response.( Granier, C., Gey, A., Roncelin, S., Weiss, L., Paillaud, E., & Tartour, E. (2021). Immunotherapy in older patients with cancer. biomedical journal, 44(3), 260-271. )

According to the invention, the biological sample is a biological sample as defined above.

According to the invention, the subject is as defined above.

In the present "treatment" means, for example, a medical treatment, e.g. allopathic, involving the intake of therapeutic agent, molecules, for example. chemical molecules, for example. molecules obtained by organic synthesis, molecules of biological origin, for example proteins, molecules originating from living organisms, for example mammals, microorganisms, plants and/or synthesized by living organisms, for example proteins, nucleic acid molecules. It can be a medical treatment, e.g. allopathic, involving the intake of therapeutic agent.

According to the invention, the therapeutic agent is as defined above.

According to the invention, the determination of the amount of $CD57^+$ EMRA $CD8^+$ T cells; $CD57^+$ $GRZB^+$ EMRA $CD8^+$ T and $CD8^+$ T cells in a biological sample is as defined above.

According to the invention, the biological sample is as defined above.

In the present the determination in a biological sample of the amount of $CD57^+$ EMRA $CD8^+$ T cells and/or $CD57^+$ $GRZB^+$ EMRA $CD8^+$ T, and $CD8^+$ T cells before treatment of a subject in step A. can be performed in a period of from 1 to 30 days before treatment, for example from 1 hour to 7 days before treatment.

In the present the determination in a biological sample of the amount of CD57$^+$ EMRA CD8$^+$ T cells and/or CD57$^+$ GRZB$^+$ EMRA CD8$^+$ T, and CD8$^+$ T cells after treatment of a subject in step B. can be performed in a period of from 1 to 30 days after treatment, for example from 1 hour to 7 days after treatment.

Advantageously, the present invention makes it possible to detect whether a treatment and/or a therapeutic agent induce immunosenescense in a subject, for example a patient, with greater sensitivity and specificity than known methods.

The method for determining the sensitivity of a condition, preferably cancer, to a therapeutic agent may be a computer-implemented methods, which involves the use of tools commonly used in this technical field, as for example, a record component, notably for recording, for each sample, the amount of CD57$^+$ EMRA CD8$^+$ T cells, CD8$^+$ T cell and/or CD57$^+$ GRZB$^+$ EMRA CD8$^+$ T cells, a storage component for storing data, a computer processor for processing data, wherein the computer processor is coupled to the storage component and configured to execute the instructions stored in the storage component in order to receive data and analyze them according to one or more algorithms and/or to calculate P1 and/or P2 value, and a display component for displaying information regarding the sensitivity of a condition, preferably cancer, to a therapeutic agent.

The method for detecting immune ageing and/or immunosenescense may be a computer-implemented methods, which involves the use of tools commonly used in this technical field, as for example, a record component, notably for recording, for each sample, the amount of CD57$^+$ EMRA CD8$^+$ T cells, CD8$^+$ T cell and/or CD57$^+$ GRZB$^+$ EMRA CD8$^+$ T cells, a storage component for storing data, a computer processor for processing data, wherein the computer processor is coupled to the storage component and configured to execute the instructions stored in the storage component in order to receive data and analyze them according to one or more algorithms and/or to calculate P3 and/or P4 value, and a display component for displaying information regarding the sensitivity of a condition, preferably cancer, to a therapeutic agent.

The method for determining the sensitivity of a condition, preferably cancer, to a therapeutic agent may be a computer-implemented methods, which involves the use of tools commonly used in this technical field, as for example, a record component, notably for recording, for each sample, the amount of CD57$^+$ EMRA CD8$^+$ T cells, CD8$^+$ T cell and/or CD57$^+$ GRZB$^+$ EMRA CD8$^+$ T cells, a storage component for storing data, a computer processor for processing data, wherein the computer processor is coupled to the storage component and configured to execute the instructions stored in the storage component in order to receive data and analyze them according to one or more algorithms and/or to calculate P1 and/or P2 value, and a display component for displaying information regarding the sensitivity of a condition, preferably cancer, to a therapeutic agent.

Another object of the invention is a device for implementing a method of the invention, comprising at least one mean for detecting and/or quantifying CD8$^+$ T cells and at least one mean for detecting and/or quantifying CD57$^+$ EMRA CD8$^+$ T cells. According to the invention, the device can further comprise detecting agents for detecting and/or quantifying CD57+ GRZB+ EMRA CD8+ T cells.

In the present, the mean for detecting and/or quantifying CD8$^+$ T cells may be a detecting agent of CD8$^+$ T cells and/or methods for detecting and/or quantifying protein biomarkers and/or cells according to the invention may be as defined above.

In the present, the mean for detecting and/or quantifying CD57$^+$ EMRA CD8$^+$ cells may be detecting agent of CD57$^+$ EMRA CD8$^+$ cells and/or methods for detecting and/or quantifying protein biomarkers and/or cells according to the invention may be as defined above.

In the present, the mean for detecting and/or quantifying CD57+ GRZB+ EMRA CD8+ T cells may be a detecting agent of CD57+ GRZB+ EMRA CD8+ T cells and/or methods for detecting and/or quantifying protein biomarkers and/or cells according to the invention may be as defined above

Another object of the invention is a kit for implementing the method of the invention comprising at least one detecting agent of CD8$^+$ T cells and at least one detecting agent of CD57$^+$ EMRA CD8$^+$ T cells. According to the invention, the kit can further comprise at least one detecting agent CD57+ GRZB+ EMRA CD8+ T cells.

In the present, the detecting agent of CD8$^+$ T cells may be as defined above. It may be for example anti-CD8 antibody, optionally with anti-T(CD3+) antibody, as defined above.

In the present, the detecting agent of CD57$^+$ EMRA CD8$^+$ cells may be as defined above. It may be for example an anti-CD57 antibody, anti-CD45RA antibody, anti-CD62L antibody and anti-CD28 antibody and anti-CD27 antibodies or any combination thereof.

In the present, the detecting agent of CD57+ GRZB+ EMRA CD8+ T cells may be as defined above. It may be for example antibodies selected from the group comprising anti-CD57 antibody, anti-CD45RA antibody, anti-CD62L antibody, anti-CCR7 antibody, anti-CD27 antibody, anti-CD28 antibody or any combination thereof, preferably anti-CD57 antibody, anti-CD45RA antibody, anti-CD27 antibody, anti-CD28 antibody

## BRIEF DESCRIPTION OF THE DRAWINGS

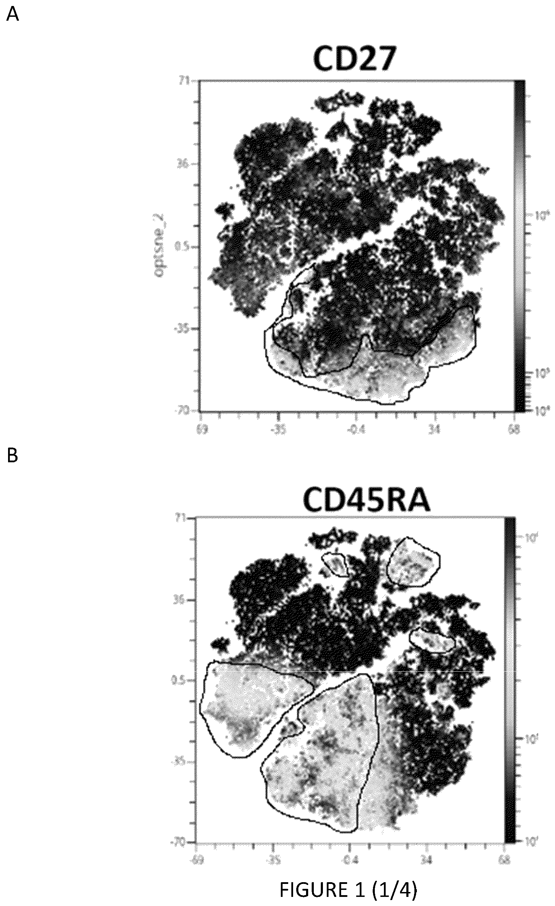

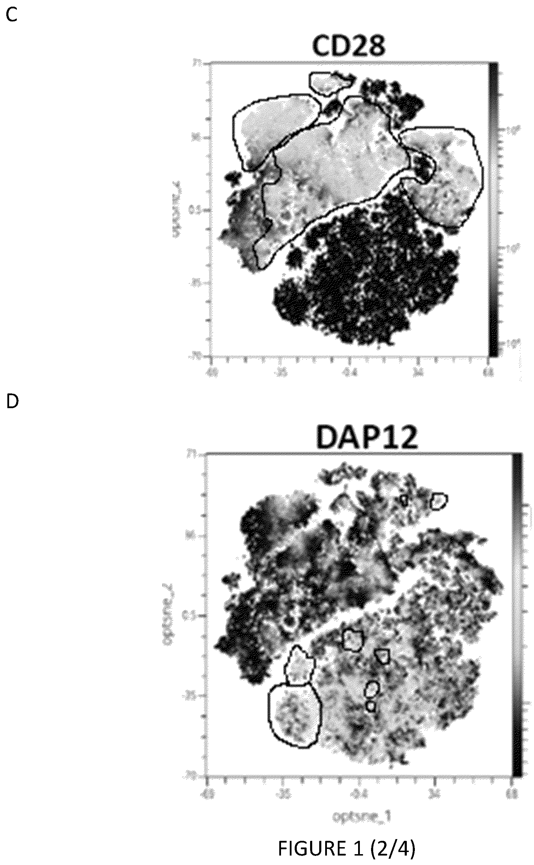

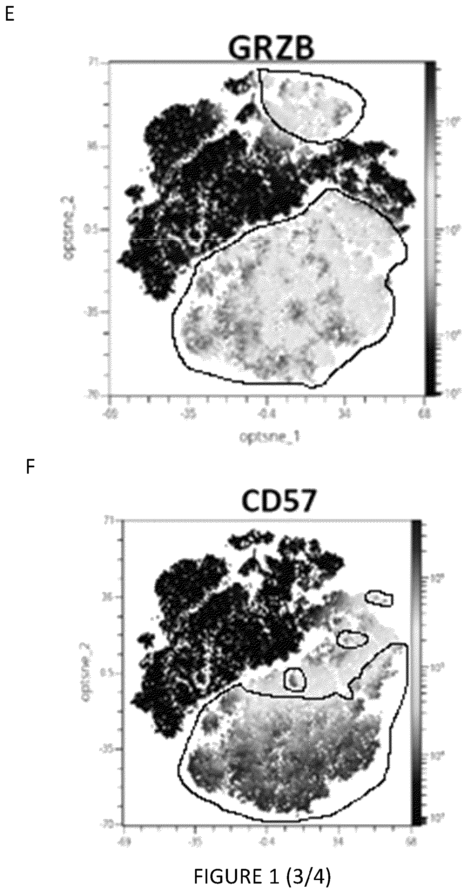

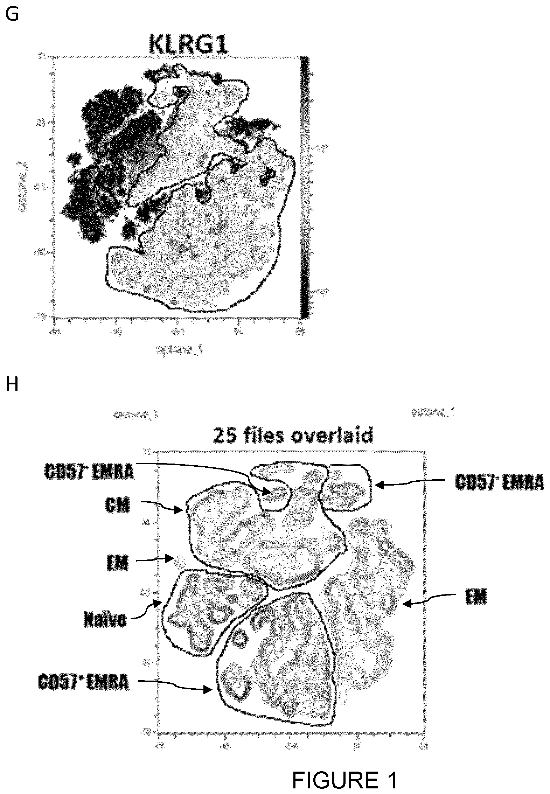

**Figure 1** The figure presents opt-SNE (optimized t-distributed Stochastic Neighbor Embedding) projections of CD8[+] T cell subsets from 25 overlaid samples with overlaid to illustrate the expression of selected markers. Each point in the plot represents a single cell, and cells with similar marker expression profiles are positioned close to each other in the 2D space. X-axis represent the optSNE_1 (first component of the projection) and y-axis the optSNE_2 (second component). They are both outputs from a single opt-SNE analysis, and together they allow for a meaningful 2D visualization of complex cellular phenotypes. The major CD8[+] T cell subpopulations are annotated based on their phenotypic clustering:

- NAIVE: Located in the central lower-left region, these cells are typically CD45RA[+] CD27[+] CD28[+] and represent antigen-inexperienced T cells.
- CM (Central Memory): Found in the upper left quadrant, these express markers such as CD45RO[+] CD27[+] CD28[+].
- EM (Effector Memory): Situated in the mid-right region, these cells show immediate effector function upon antigen encounter.
- EMRA CD57[-] and EMRA CD57[+] (Effector Memory RA re-expressing): These subsets are located in the right top and the lower half of the plot, with CD57[+] cells indicating terminal differentiation and senescence, while CD57[-] cells represent less differentiated EMRA cells.In the scatterplots, marker expressions is represented by a continuous black to grey with gradient. In this figure, a dimension reduction of the cells according to their expression of all the markers (CD57, KLRG1, CD27, CD28, CD45RA, GRZB and DAP12) is represented according to a given marker (for instance CD27), with optimized t-distributed stochastic neighbor embedding (optSNE) as x-axis and y-axis. Figure 1A represents the scatterplots for cells expressing CD27 wherein the zone with expression levels greater than $4.10^5$ is circled. Figure 1B represents the scatterplots of cells expressing CD45RA, where the zones with expression levels greater than $2.10^5$ are circled. Figure 1C represents the scatterplots of cells expressing CD28, the zones with expression levels greater than $1.10^5$ are circled. Figure 1D represents the scatterplots of cells expressing DAP12, the zones with expression levels greater than $5.10^4$ are circled. Figure 1E represents the scatterplots of cells expressing GRZB, the zones with expression levels greater than $1.10^5$ are circled. Figure 1F represents the scatterplots of cells expressing CD57, the zones with expression levels greater than $1.10^6$ are circled. Figure 1G represents the scatterplots of cells expressing KLRG1, the zone with expression levels greater than $4.10^4$ is circled. Figure 1H represent the results of an optSNE1/optSNE2 plot that reveals 5 distinct clusters corresponding to the differentiation stages, identified through CD27, CD28, CD45RA expression levels as naïve, central memory (CM), effector memory (EM) and terminally differentiated effector memory (EMRA). The expression of NK receptors (CD57 KLRG1) and granzyme B functional marker expressions characterize mainly effector memory cells (EM and EMRA). DAP12 is highly expressed in CD57hi CD8+ T cells. Granzyme B+ CD8+ T cells are mainly CD28-.

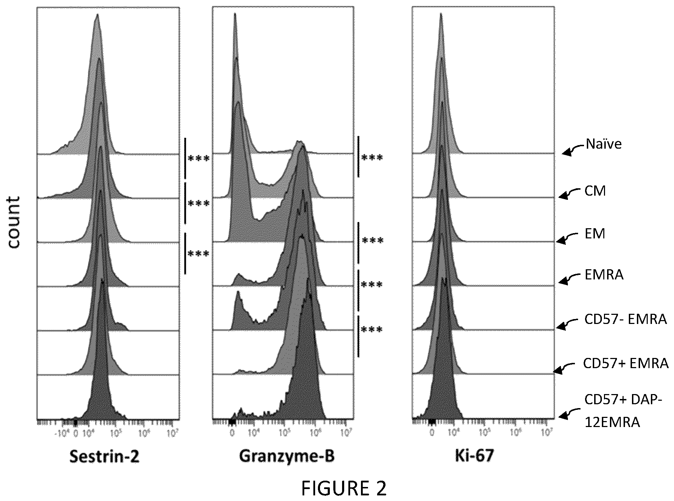

**Figure 2** is a diagram representing the mean fluorescence intensity of Sestrin-2, granzyme B, and Ki-67 across CD8+ T cells maturation stages i.e. in naïve, central memory (CM), effector memory (EM), terminally differentiated effector memory (EMRA), terminally differentiated effector memory CD57[-] (CD57- EMRA), terminally differentiated effector memory CD57[+] (CD57+ EMRA) and terminally differentiated effector memory CD57[+] DAP-12 cells (CD57+ DAP-12 EMRA). In other words, the mean fluorescence intensity (MFI) measured in count (ordinate) of Sestrin-2, granzyme B, and Ki-67 was measured across different stages of CD8+ T cell maturation, ranging from the least differentiated (naïve) to the most differentiated (CD57+ DAP12+ EMRA) cells (n=35. Differences between the two groups were tested using a two-sided unpaired t-test. The p-values are indicated by asterisks as follows: <0.05*, <0.01**, <0.001***, <0.0001****.

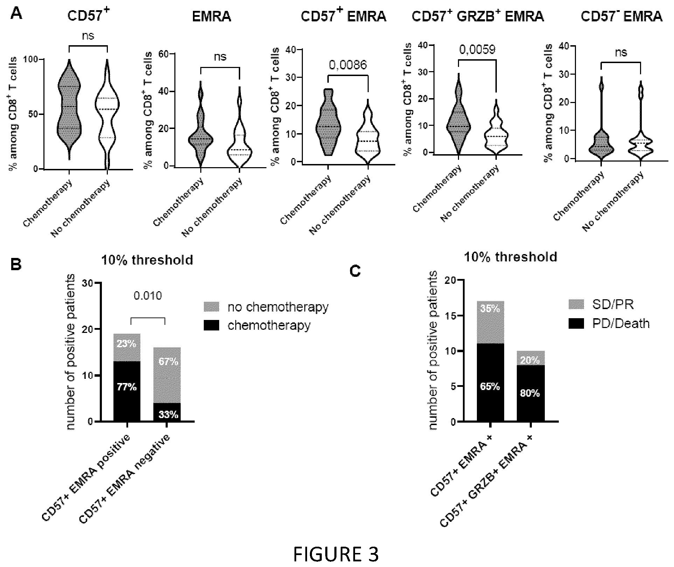

**Figure 3** represents the results regarding the association of CD8+ T cells senescence with prior chemotherapy and response to anti-PD-1/PD-L1 therapy. Figure 3A represents the percentage among CD8+ T cells (ordinate) in the sample of CD8+ T cells expressing CD57 (CD57[+]), EMRA, CD57 and EMRA (CD57[+] EMRA), CD57, granzyme B and EMRA (CD57[+] GRZB[+] EMRA) or EMRA and not CD57 (CD57[-] EMRA) from a subject treated with chemotherapy or not (No chemotherapy). In other words, this figure represents the results regarding the senescence of CD8+ T cells, indicated by EMRA, CD57, and granzyme B (GRZB) expression in relation to prior chemotherapy exposure. The data are presented as a proportion of the total CD8+ T cell population. Figure 3B is a histogram representing the number of positive patient (abscissa) with or not CD57 and EMRA (CD57[+] EMRA) cells in biological sample having or not a chemotherapy. In other words, this figure represents the results of frequency of CD57+ EMRA CD8+ T cells (cut-off 10%) is compared based on pre-exposure to chemotherapy. Figure 3 C is a histogram representing the number of positive patient (abscissa) comprising CD57+ EMRA CD8+ T cells (CD57+ EMRA+) or CD57+ GRZB+ EMRA CD8+ T cells (CD57+ GRZB+ EMRA+) in biological sample from patient regarding the treatment response: progressive disease (PD), partial response (PR), stable disease (SD) or death. In others words, Figure 3 C represents the proportion of CD57+ EMRA and CD57+ GRZB+ EMRA CD8+ T cells (cut-off 10%) in relation to treatment response, categorized as progressive disease (PD), partial response (PR), or stable disease (SD).

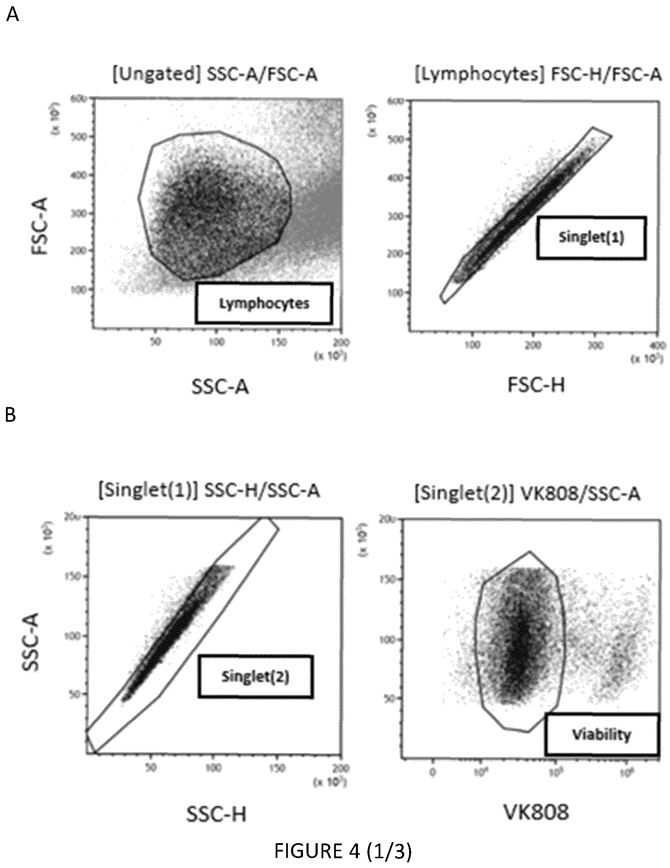

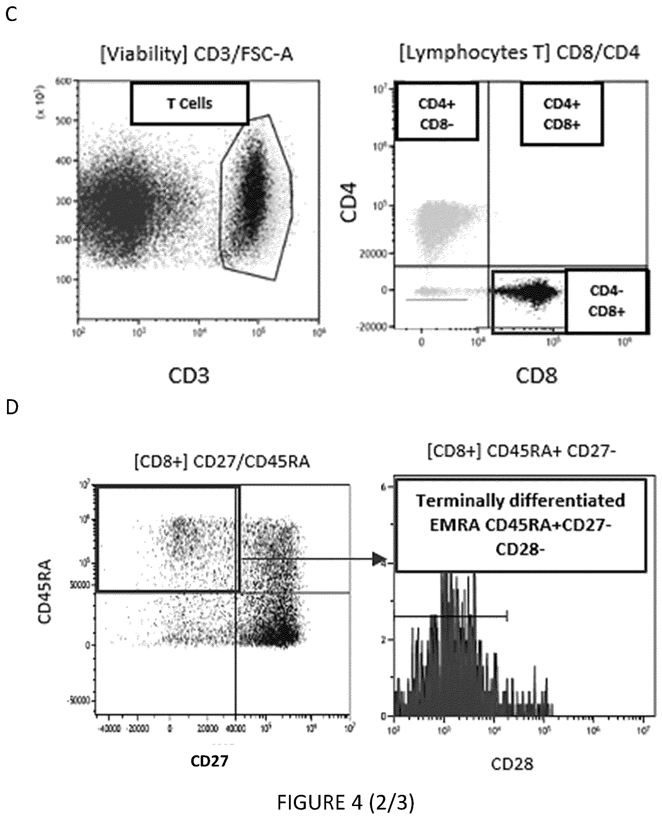

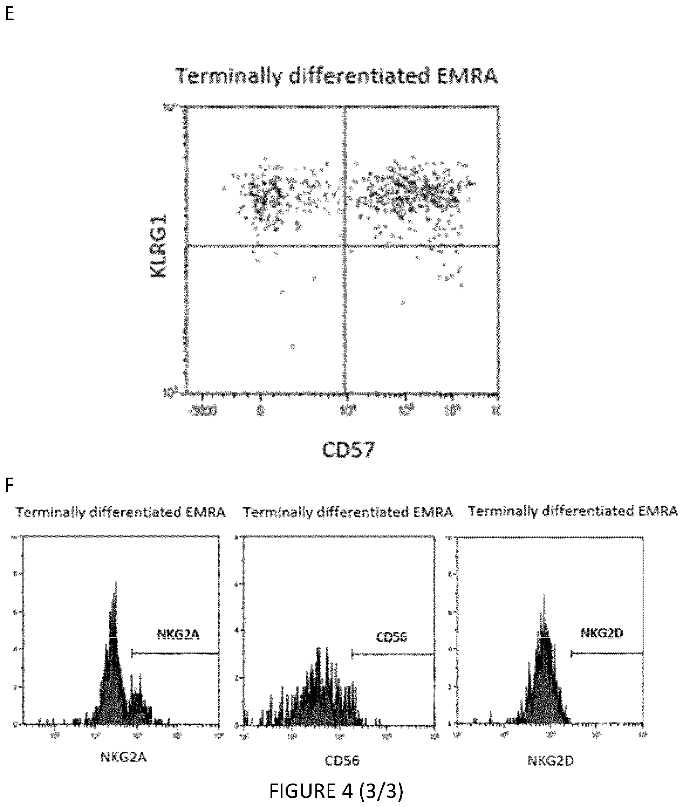

**Figure 4** represents the gating strategy for conventional analysis. Lymphocytes were first gated based on scatter parameters, followed by selection of viable (viability dye VK808 negative) and CD3+/CD8+/CD4- cells. Doublets were excluded from the analysis. The most fully differentiated T cells were identified by gating on CD45RA+ CD27-CD28- cells. The expression levels of key markers, including CD57, KLRG1, NKG2A, CD56, and NKG2D, were then analysed among total CD8+ T cells and terminally differentiated (EMRA) CD8+ T cells. In figures 4A, 4B, 4C, 4D and 4E, the cells are regrouped depending on the expression of the markers mentioned in the x and y axis (unit: fluorescence intensity). In Figure 4F, the cells are regrouped depending on the marker mentioned in the x axis (unit: fluorescence intensity) and the number of event in y axis (unit: number of cell). A forward scatter height (FSC-H) *versus* forward scatter area (FSC-A) density plot is used to exclude doublets and isolate singlets as shown in Figure 4A (right panel). A side scatter height (SSC-H) *versus* side scatter area (SSC-A) density plot is used to exclude doublets and isolate singlets as shown in Figure 4B (left panel). Figure 4 A shows 2 flow cytometry dot plots representing: (i) on the left panel, the cells by forward scatter (FSC) (y axis) and side scatter (the SSC) (x axis) of the cells in the sample and, (ii) on the right panel, it represents the Lymphocytes (isolated on the left panel gating), according to FSC-H in x axis and FSC-A in the y axis, in order to identify the singlets and eliminate the doublets.. Figure 4 B shows, (i) on the left panel a flow cytometry image of the Singlet 1, (identified by the gating on Figure 4A) according to SSC-H in x axis and SSC-A in y axis and (ii) on the right panel the singlets (identified by the gating on the left panel of the Figure 4B) according to the viability dye ViaKrome 808 (VK808) in x axis and the SSC-A in y axis, allowing the identification of the viable cells (by the negative expression of the viability dey VK808). Figure 4 C shows 2 flow cytometry dot plots representing: (i) on the left panel, the cells by forward scatter (FSC-A) (y axis) and CD3 (x axis) of the previously identified viable cells in Figure 4B, (ii) on the right panel, the CD3+ lymphocytes, named T cells, according to their CD4 (y axis) and CD8 (x axis) expression, allowing the identification of the CD8+ Lymphocytes (that express CD8 and do not express CD4). Figure 4 D shows a flow cytometry dot plot and an histogram representing: (i) on the left panel, the CD8+ Lymphocytes previously identified on Figure 4C according to CD45RA (y axis) and CD27 (x axis) allowing the identification of the EMRA that express CD45RA and do not express CD27 and (ii) in the right panel, the terminally differentiated EMRA thanks to the absence of CD8 expression, represented in the x axis, with the number of events in the y axis. Figure 4 E shows a flow cytometry dot plot representing the terminally differentiated EMRA identified on Figure 4D accrding to CD57 (x axis) and KLRG1 (y axis). The Figure 4 F shows 3 histograms representing the expression of NKG2A, CD56 and NKG2D (x axis) respectively, according to the number of events.

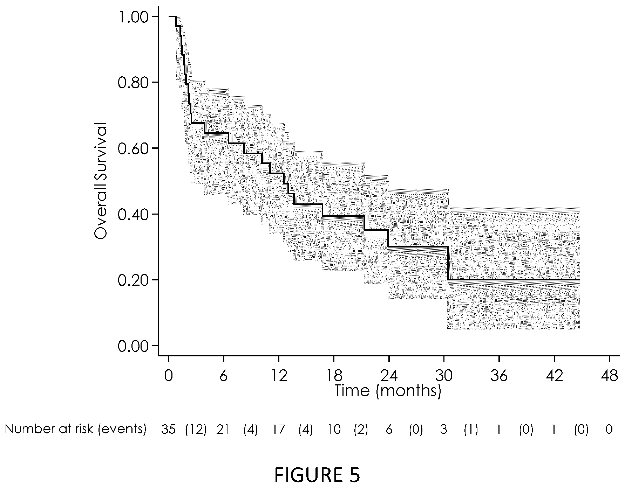

**Figure 5** represents a diagram representing the overall survival in months of the studied population (ordinate) along the time in month (abscissa). In this figure the number at risk represents the number of patients at risk of death.

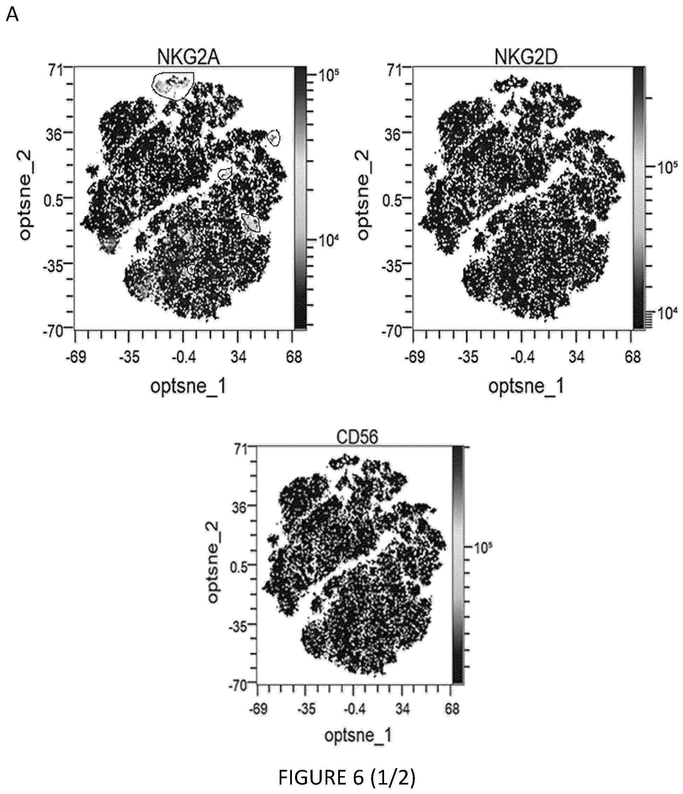

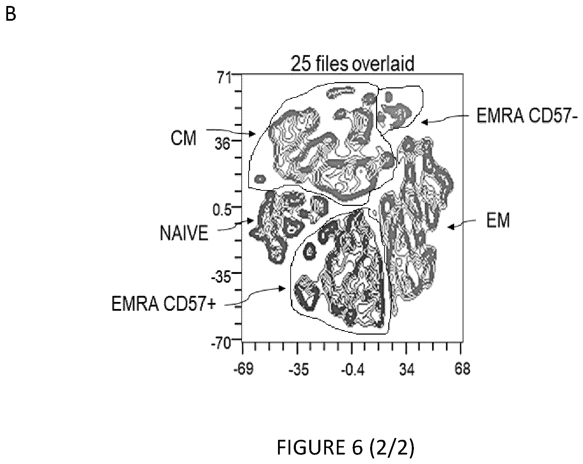

**Figure 6 Density opt-SNE plots of CD8+ T cells in the ICI-treated older cancer patient cohort.** Density opt-SNE plots of CD8+ T cells in the ICI-treated older cancer patient cohort, with data from 25 files overlaid to display the expression of selected markers. The scatterplots use a continuous color scale from blue (indicating minimal expression) to red (indicating maximal expression).

Figure 6A showcases the opt-SNE plots highlighting the expression of NKG2D and NKG2A. In the case of the NKG2A scatterplot, the zones where the expression level is greater than $2.10^4$ are circled. No zone appears on the NKG2D scatterplot where the expression level is greater than $2.10^4$.

Figure 6B: The figure presents opt-SNE (optimized t-distributed Stochastic Neighbor Embedding) projections of CD8+ T cell subsets from 25 overlaid samples. Each point in the plot represents a single cell, and cells with similar marker expression profiles are positioned close to each other in the 2D space.X-axis represent the optSNE_1 (first component of the projection) and y-axis the optSNE_2 (second component). They are both outputs from a single opt-SNE analysis, and together they allow for a meaningful 2D visualization of complex cellular phenotypes. The results of opt-SNE plots that reveals 5 distinct clusters corresponding to the differentiation stages, identified through CD27, CD28, CD45RA expression levels as naïve, central memory (CM), effector memory (EM) and terminally differentiated effector memory (Effector Memory RA re-expressing (EMRA)). The major CD8+ T cell subpopulations are annotated based on their phenotypic clustering:

- NAIVE: Located in the central lower-left region, these cells are typically CD45RA$^+$ CD27$^+$ CD28$^+$ and represent antigen-inexperienced T cells.
- CM: Found in the upper left quadrant, these express markers such as CD45RO$^+$ CD27$^+$ CD28$^+$.
- EM: Situated in the mid-right region, these cells show immediate effector function upon antigen encounter.
- EMRA CD57$^-$ and EMRA CD57$^+$: These subsets are located in the lower half and the right top of the plot, with CD57$^+$ cells indicating terminal differentiation and senescence, while CD57$^-$ cells represent less differentiated EMRA cells. The expression of NK receptors (CD57 KLRG1) characterizes mainly effector memory cells (EM and EMRA).

## EXAMPLES

**Example 1: CD57$^+$ EMRA CD8$^+$ T cells in cancer patients over 70: Associations with prior chemotherapy and response to anti-PD-1/PD-L1 therapy**

List of abbreviations

ADL Activities of Daily Living
ALK Anaplastic Lymphoma Kinase
BMI Body Mass Index
CBC Complete Blood Count
CGA Comprehensive Geriatric Assessment
CIRS-G Cumulative Illness Rating Scale-Geriatric
CKD Chronic Kidney Disease
CI Confidence Interval
CR Complete Response
CRP C-Reactive Protein
ECOG-PS Eastern Cooperative Oncology Group Performance Status
ELCAPA ELderly CAncer PAtient
EMRA Effector Memory Re-expressing CD45RA
ERK Extracellular signal-Regulated Kinase
EMRA Effector Memory Re-expressing CD45RA
HAS French National Authority for Health
IADL Instrumental Activities of Daily Living
FMO Fluorescence Minus One
FoxP3 Forkhead Box P3
ICI Immune Checkpoint Inhibitors
JNK c-Jun N-terminal Kinases
KAR NK cell activating receptors
KIR NK cell inhibitory receptors
KLRG1 Killer Cell Lectin-like Receptor G1
LDH Lactate Dehydrogenase
MAPK Mitogen-activated protein kinases
Mini-GDS Mini-Geriatric Depression Scale
MMSE Mini-Mental State Examination
NKG2A Natural Killer Group 2A receptor
NKG2D Natural Killer Group 2D receptor
NHL Non-Hodgkin lymphoma
NK cells Natural Killer cells
NLR Neutrophil-to-Lymphocyte Ratio
optSNE Optimized t-distributed stochastic neighbor embedding
OR Odds Ratio / aOR Adjusted Odds Ratio
ORR Overall Response Rate
OS Overall Survival
PBMC Peripheral Blood Mononuclear Cells
PD-1 Programmed Cell Death Protein 1
PD-L1 Programmed Death-Ligand 1

PR Partial Response
Ses-2 Sestrin-2
SIP Senescence Immunological Profile
TCR T cell receptor
TMB Tumour Mutational Burden

Immune ageing complicates cancer treatment in older individuals. While immunotherapy targeting the PD-1/PD-L1 pathway can reinvigorate T cells, these cells tend to become senescent with age. CD8+ T cell subsets usually associated with senescence, in cancer patients over 70 years old who are undergoing anti-PD-1/PD-L1 immunotherapy, was studied and the relationship between these senescent cells and prior chemotherapy exposure was determined. The analyzed data were from the Elderly Cancer Patient (ELCAPA) cohort, which included 35 patients.

**AI Materials and methods**

**2.1. Study design**

2.1.1. Patients

Analyzed data were from the ELderly CAncer PAtient (ELCAPA) cohort study. ELCAPA is a French, prospective observational, multicenter cohort of patients aged 70 years or older with newly diagnosed cancer at any stage. All patients included in the ELCAPA study were referred to a geriatrician for a Comprehensive Geriatric Assessment (CGA) prior to treatment initiation, at one of 19 centers in the Ile-de-France region (Caillet P, Canoui-Poitrine F, Vouriot J, Berle M, Reinald N, Krypciak S, et al. Comprehensive Geriatric Assessment in the Decision-Making Process in Elderly Patients With Cancer: ELCAPA Study. JCO. 2011;29:3636-42 [16]).

For the present study, patients with an ICI treatment intent and a peripheral blood mononuclear cells (PBMC) blood sample available were included. These patients were enrolled in the ELCAPA cohort at two centers: Georges Pompidou European Hospital and Cochin Hospital, Paris, France. Blood samples were collected just before the initiation of ICI treatment, regardless of the previous treatment line.

The immunotherapy agents used for cancer treatment included anti-PD1: pembrolizumab (MSD), nivolumab (bristol myers squibb) and anti-PD-L1: durvalumab (AstraZeneca), avelumab (Pfizer Merck). Prior chemotherapy regimens included combinations of carboplatin and taxol, carboplatin and gemcitabin, or taxol alone.

Of the 44 patients originally enrolled in this study, 35 were eligible for cytometry analysis.

The ELCAPA study protocol received approval from the institutional ethical committee (*CPP Ile-de-France I,* Paris, France; reference: 2019 mai-MS121) and was registered on ClinicalTrials.gov (NCT: 02884375). All patients were provided with detailed information about the study's objectives and procedures and gave verbal consent for the clinical aspects and written informed consent for the biological sample analysis.

2.1.2. Endpoints

Tumor response was evaluated by RECIST v1.1 criteria (Tazdait M, Mezquita L, Lahmar J, Ferrara R, Bidault F, Ammari S, et al. Patterns of responses in metastatic NSCLC during PD-1 or PDL-1 inhibitor therapy: Comparison of RECIST 1.1, irRECIST and iRECIST criteria. European Journal of Cancer. 2018;88:38-47 [17]) and classified in complete response (CR), partial response (PR), stable disease (SD), or progressive disease (PD). The primary endpoint is the Overall Response Rate (ORR) at 6 months, defined as CR, PR, or SD at 6 months, *versus* non-response, defined as PD at 6 months, or death within 6 months. Secondary endpoints were time until death (overall survival) at 3 and 6 months. The index date was the date of CGA.

2.1.3. Clinical and biological characteristics

2.1.3.1. Clinical characteristics

Demographic variables (age and sex) and oncological variables (primary tumour site and metastatic status) were collected at inclusion. The global functional status was assessed using the Eastern Cooperative Oncology Group Performance Status (ECOG-PS) scale [18]. ECOG-PS was categorized into three levels: 0-1 (fully active or restricted in strenuous activity) and 2, and 3-4 (increasing disability).

The CGA at inclusion, performed by a geriatrician with expertise in oncology, included several parameters: autonomy (activities of daily living (ADL (Katz, 6 items)) score, abnormal if score ≤5/6; instrumental ADL (IADL (Lawton, 8 items)) score, abnormal if score ≤7/8), mobility and fall risk (timed get up and go test, abnormal if unable to perform, or time to perform >20s), nutritional status (body mass index in kg/m$^2$ (BMI); weight loss ≥10% in the last 6 months), cognitive status (mini-mental state examination (MMSE), abnormal is score ≤24/30), mood (mini geriatric depression scale (GdS) score, risk for depression if score ≥1/4), and comorbidities (cumulative illness rating scale for geriatrics (CIRS-G), 5-point scale ranging from 0 (no dysfunction) to 4 (extremely severe dysfunction)) (. Caillet P, Canoui-Poitrine F, Vouriot J, Berle M, Reinald N, Krypciak S, et al. Comprehensive Geriatric Assessment in the Decision-Making Process in Elderly Patients With Cancer: ELCAPA Study. JCO. 2011;29:3636-42 [16]).

### 2.1.3.2. Biological characteristics

Routine laboratory tests, including lactate dehydrogenase (LDH), complete blood count (CBC), C-reactive protein (CRP) testing were performed at the centre of inclusion and extracted from a secured and standardized electronic case report. LDH and CRP were measured on the AU5810 analyzer according to the manufacturer's instructions (Beckman Coulter®) and CBC on the XN according to the manufacturer's instructions (Sysmex).

## 2.2. Characterization of T cell senescence by flow cytometry

### 2.2.1. Experiment protocol

Senescence phenotyping of CD8$^+$ T cells was performed on the biological samples provided by the Biological Resources Center and Tumor Bank Platform (BB-0033-00063). Mononuclear cells were separated using a Ficoll density gradient thanks to Leucosep™ tubes according to the instructions of the manufacturer (Greiner Bio-One) and a numeration was performed. Five to ten million mononuclear cells were frozen per vial in 1 mL of a serum-free medium containing 10% DMSO (CryoMaxx, PAA, Pasching, Austria). After homogenization, the cryotubes were placed in a freezing container (Nalgene Mr. Frosty, Thermo Fisher Scientific, MA, USA). The samples were stored overnight at -80°C, and the frozen vials were then transferred to liquid nitrogen until further analysis. The frozen human PBMC were thawed and incubated with flow cytometry antibodies. Cells were stained with a fixable viability stain and labelled for surface (CD3, CD4, CD8, CD27, CD45RA, CD28, CD57, KLRG1, CD56, NKG2A, NKG2D) and intracellular (granzyme-B (GRZB), Ki-67, Sestrin-2, and Dap-12) markers (see Table 1 for markers, antibodies, clones and brands). All staining except for sestrin-2, was direct. Sestrin-2 expression was assessed after fixation and permeabilization (BioLegend FoxP3 Perm and Fix). After labelling, red blood cells were lysed using VersaLyse lysing solution (Beckman Coulter, Inc., USA) according to the manufacturer's recommendations. Surface and intracellular markers were analyzed by flow cytometry using a Navios flow cytometer (Beckman Coulter, Inc., USA). Calibration of the cytometer was performed daily with Flow-Set and Flow-Check fluorosphere (Beckman Coulter, Inc., USA). Fluorescence minus one (FMO) controls were used to verify the absence of spillover after applying the compensation matrix and as gating controls. Additionally, isotype controls were used for Sestin-2, Dap-12, GRZB, NKG2A and NKG2D.

**Table 1: Antibodies used for T cell senescence staining**

| Target | Fluorochrome | Clone | Supplier | Reference |
|---|---|---|---|---|
| Viability | ViaKrome 808 | | Beckman | C36628 |
| CD3 | BUV395 | UCHT1 | BD | 563546 |
| CD4 | PC7 | SFCI12T4 D11 | Beckman | 737660 |
| CD8 | BV786 | RPA-T8 | BD | 563823 |
| CD27 | BB700 | L128 | BD | 746084 |
| CD45RA | bv421 | HI100 | BIOLEGEND | 304130 |
| CD28 | PE/CY5 | CD28.2 | BD | 555730 |
| CD57 | PE/CF 594 | NK-1 | BD | 562488 |
| KLRG1 | ALEXA 488 | 13F12F2 | INVITROGEN | 53-9488-42 |
| CD56 | APC-R700 | NCAM16-2 | BD | 565139 |
| NKG2A | BV510 | 131411 | BD | 747922 |
| NKG2D | BV650 | 1d11 | BD | 563408 |
| Granzyme B | PE | GB11 | BD | 561142 |
| Ki-67 | BV480 | B56 | BD | 566109 |

(continued)

| Target | Fluorochrome | Clone | Supplier | Reference |
|--------|--------------|-------|----------|-----------|
| Dap-12 | AF647 | 406288 | BD | 566603 |
| Sestrin-2 | AF700 | D1B6 | CST | 8487 |

2.2.2. Gating strategy and T cell differentiation identification

Singlet & viable cells were selected before analyzing cell subsets. This experiment focused on CD8$^+$ T cells. CD4$^-$ CD8$^+$ CD3$^+$ cells were selected. CD27, CD28, and CD45RA were employed to classify the maturational stages of CD8$^+$ T cells. Naïve T cells express CD45RA while memory T cells are CD45RA$^-$ (CD45RO$^+$). Naïve and central memory T cells coexpress the costimulatory molecules CD27 and CD28, while effector memory T cells are predominantly CD28$^-$ and CD27$^-$. The most differentiated T cells reexpress CD45RA [9,10]. CD45RA$^+$CD27$^+$ cells were considered as naïve, CD45RA$^-$CD27$^+$ as central memory (CM) and CD45RA$^-$CD27$^-$ as effector memory (EM). As CD8$^+$ T cell tend to lose CD28 before losing CD27 [19], CD27$^-$CD45RA$^+$ CD8+ T cell were mainly CD28$^-$ (Figure 4). CD27$^-$CD28$^-$CD45RA$^+$, the most differentiated effector CD8$^+$ T cell subset, was referred to as EMRA in the present example (Koch S, Larbi A, Derhovanessian E, Ozcelik D, Naumova E, Pawelec G. Multiparameter flow cytometric analysis of CD4 and CD8 T cell subsets in young and old people. Immun Ageing. 2008;5:1-12 [8] Romero P, Zippelius A, Kurth I, Pittet MJ, Touvrey C, Iancu EM, et al. Four Functionally Distinct Populations of Human Effector-Memory CD8 + T Lymphocytes. J Immunol. 2007;178:4112-9 [9])

CD57, CD56, NKG2A, NKG2D, Sestrin-2, and Dap-12 were used to assess senescence, while the cytotoxicity of GRZB was also investigated. The results were studied in percentage of these markers among total CD8$^+$ T cells. Absolute count was not available. The gating strategy is shown in Figure 4. In particular, Figure 4 illustrates the detection and characterization of senescent CD8$^+$ T cells using flow cytometry. The gating strategy is detailed as follows. First, lymphocytes were identified among PBMCs based on their size (forward scatter height) and granularity (side scatter height). Doublets and other artifacts were excluded by selecting singlets using forward scatter height (FSC-H) versus forward scatter area (FSC-A), and side scatter height (SSC-H) versus side scatter area (SSC-A) density plots.

Viable cells were then selected by excluding those positive for the viability dye, which stains dead cells by binding to free amine residues. Among viable cells, CD3$^+$ lymphocytes-corresponding to T cells-were identified. CD4$^+$ and CD8$^+$ T cell subsets were subsequently distinguished.

The CD8$^+$ T cells were further analyzed for:

(i) their differentiation status, with EMRA cells (CD45RA$^+$ CD27$^-$ CD28$^-$) representing the most differentiated-and thus senescent-subset, and
(ii) the expression of senescence-associated markers, including CD57, KLRG1, NKG2A, CD56, and NKG2D.

**2.3 Bioinformatics and statistical analysis**

*Unsupervised analysis by opt-SNE*

Flow cytometry data were extracted from Kaluza Flow Cytometry Software v2.2 (Beckman Coulter).

Dimensionality reduction of flow cytometry data was performed using the opt-SNE, an optimized t-SNE algorithm, a modified version of t-SNE (t-distributed stochastic neighbor embedding) (Belkina AC, Ciccolella CO, Anno R, Halpert R, Spidlen J, Snyder-Cappione JE. Automated optimized parameters for T-distributed stochastic neighbor embedding improve visualization and analysis of large datasets. Nat Commun. 2019;10:5415 [20]) on the OMIQ (Domatics) platform.

After establishing the compensation matrix, CD3$^+$CD8$^+$CD4$^-$ T cells were extracted and an arcsinh transformation was applied. After a preliminary cleaning step was conducted, data from 25 out of 35 patients were suitable for analysis. The OptSNE algorithm was therefore applied to 25 patients, with analysis performed on a range of 1500 to 3000 CD3$^+$ cells per sample. This subsample was merged for analysis. Identification of the target population was conducted based on a two-dimensional representation, and patients excluded during the cleaning step were recovered using the classical gating method.

CD27, CD45RA, CD28, CD57, KLRG1, CD56, NKG2A, NKG2D, GZRB, Ki-67, Sestrin-2 and Dap-12 were integrated in the algorithm, with the following setup for max iterations: 1000, optSNE end: 5000, perplexity: 30, theta: 0.5, components: 2, random seed: 4478, verbosity: 25.

*Statistical analysis*

Descriptive statistics included numbers (n) (%), mean $\pm$ SD (standard deviation), or median interquartile range (IQR) [1st quartile - 3rd quartile] for baseline clinical and biological characteristics. Comparison between groups was conducted using Pearson's Chi-square test, Fischer, or Student T-test, Wilcoxon as appropriate. ORR was expressed as n (%) at 6 months. Overall survival was estimated by using the Kaplan-Meier curve.

A paired Wilcoxon signed rank test was performed to compare the median fluorescence intensity of ses-2, granzyme B and Ki-67 between differentiation stage types, taking into account the clustered structure of the data. P values from the Wilcoxon signed rank test were corrected for multiple comparisons using the false discovery rate method. The association between CD8+ T cell senescence and response was analyzed using Firth's logistic regression due to the small sample size. This analysis was conducted in both univariate form and with adjustment for cancer site (lung *versus* other locations) and prior chemotherapy. No imputation was performed for missing data. The significance threshold was set at 0.05, and all tests were two-tailed.

Statistical analysis was performed by using Stata SE v17.0 (College Station, TX, USA).

## B/ Results

### 1. Clinical characteristics (Table 1)

A total of 35 patients were included. The median follow-up was 24.9 months [95% CI: 16.8 ; 33.8]. The median age of the cohort was 79 years [74-84]. At 6 months, the overall response rate (ORR) was 36.7% (11/30), and the stable disease (SD) and partial response (PR) rate being 16.7% (5/30) and 20% (6/30), respectively [missing data: n=5] (Table 2). Overall survival (OS) at 3 and 6 months were 67.7% (CI 95%: 49.2 - 80.6) and 64.6% (CI 95%: 46.1 - 78.1), respectively (Figure 5).

The main patient characteristics are listed in Table 2. Among the 35 patients, 17 (48%) had previously received chemotherapy, while 18 (52%) received ICI as a first-line treatment (including 2 patients who received ICI in combination with chemotherapy). There was no significant difference in age between these two groups.

The male-to-female ratio tended to be lower in the chemotherapy pretreated group (p=0.060). Tumour types were mainly lung, renal, and bladder cancer, with no difference in cancer types between the two groups. At the time of study inclusion, the three patients without metastasis were all in the chemotherapy-pretreated group.

There were no differences between patients with or without prior chemotherapy before ICI treatment in functional status (ECOG-PS, ADL, IADL, TGUG), nutritional status (BMI, weight loss in the past 6 months), cognitive status, mood and comorbidity (CIRS-G). Additionally, the results of routine lab tests for LDH, lymphocytes and neutrophils count, and CRP/albumin were not different between the 2 groups (Table 2).

**Table 2: Patients characteristics for the whole population and according to first-line chemotherapy prior to anti-PD-1/PD-L1.**

| Baseline characteris tics | Data available | Total (n=35) | No chemotherapy prior to ICI (n=18) | Chemotherapy prior to ICI (n=17) | P-value |
|---|---|---|---|---|---|
| Age | 35 | 79 [74 - 84] | 78.5 [73 - 84] | 79 [77 - 84] | 0.894 |
| Sex (male) | 35 | 26 (74.3%) | 16 (88.9%) | 10 (58.8%) | 0.060 |
| Tumour type | 35 | | | | 0.097 |
| Colon | | 1 (2.9%) | 0 (0%) | 1 (5.9%) | |
| Stomach | | 1 (2.9%) | 0 (0%) | 1 (5.9%) | |
| Bladder | | 5 (14.3%) | 2 (11.1%) | 3 (17.6%) | |
| Kidney | | 5 (14.3%) | 5 (27.8%) | 0 (0%) | |
| Lung | | 21 (60.0%) | 11 (61.1%) | 10 (58.8%) | |
| ENT | | 1 (2.9%) | 0 | 1 (5.9%) | |
| Mesothelio ma | | 1 (2.9%) | 0 (0%) | 1 (5.9%) | |
| Metastases | 31 | | | | 0.081 |
| No | | 2 (6.5%) | 0 | 2 (14.3%) | |
| Yes | | 28 (90.3%) | 17 (100%) | 11 (78.6%) | |
| unknown | | 1 (3.2%) | 0 (0%) | 1 (7.1%) | |

(continued)

| Baseline characteris tics | Data available | Total (n=35) | No chemotherapy prior to ICI (n=18) | Chemotherapy prior to ICI (n=17) | P-value |
|---|---|---|---|---|---|
| ECOG-PS | 28 | | | | >0.99 |
| 0 | | 2 (7.1%) | 1 (5.9%) | 1 (9.1%) | |
| 1 | | 11 (39.3%) | 7 (41.2%) | 4 (36.4%) | |
| 2 | | 14 (50.0%) | 8 (47.1%) | 6 (54.5%) | |
| 3 | | 1 (3.6%) | 1 (5.9%) | 0 (0%) | |
| ADL score (/6) | 30 | 6 [5.5 - 6] | 6 [5.5 - 6] | 6 [5.5 - 6] | 0.816 |
| IADL score (/8) | 28 | 6.5 [3 - 8] | 5.5 [3 - 8] | 7.5 [3.5 - 8] | 0.442 |
| TUG (≤20s) | 20 | 16 (80.0%) | 11 (84.6%) | 5 (71.4%) | 0.587 |
| BMI | 28 | 23.4 [21.2-25.3] | 23.3 [20.1 - 24.5] | 24.8 [21.3 - 26.0] | 0.218 |
| Weight loss over 6 months | 28 | 7 (25.0%) | 3 (17.7%) | 4 (36.4%) | 0.381 |
| MMSE score (/30) | 17 | 27 [25 - 30] | 28 [27 - 30] | 24.5 [21.5 - 28.5] | 0.063 |
| Mini-GDS score (/4) | 25 | 0 [0 - 1] | 0 [0 - 2] | 0 [0-1] | 0.735 |
| CIRS-G | 19 | 10 [6 - 13] | 8 [2 - 16] | 10 [7 - 10] | 0.967 |
| LDH (UI/L) | 12 | 214 [190 - 235] | 202.5 [177.5 - 235] | 218 [213 - 550] | 0.307 |
| Lymphocyt e count (/mm$^3$) | 22 | 1230 [1040 - 1570] | 1295 [870 - 1570] | 1230 [1085 - 1595] | 0.710 |
| Neutrophil s (/mm$^3$) | 24 | 5940 [4070 - 7080] | 6175 [4206.5 - 7690] | 5205 [4070 - 6220] | 0.426 |
| Ratio NLR | 22 | 3.8 [2.6 - 7.5] | 6.6 [3.5 - 8.2] | 3.5 [2.5 - 4.3] | 0.166 |
| CRP | 28 | 13.9 [5.8 - 22.8] | 14.4 [5.6 - 29.7] | 12.6 [7.0 - 22.8] | 0.837 |
| Ratio CRP/ALBU | 19 | 0.37 [0.18 - 0.58] | 0.29 [0.13 - 0.58] | 0.45 [0.25 - 0.57] | 0.539 |
| ICI line | 35 | | | | NR |
| 1 | | 18 (51.4%) | 18 (100%) | 0 | |
| 2 | | 15 (42.9%) | 0 | 15 (88.2%) | |
| >2 | | 2 (5.7%) | 0 | 2 (11.8%) | |
| ICI molecule | 35 | | | | 0.034 |
| Anti-PD1 | | 23 (65.7%) | 11 (61.1%) | 12 (70.6%) | |
| Anti-PD-L1 | | 5 (14.3%) | 1 (5.6%) | 4 (23.5%) | |
| Anti-PD1 + anti-CTLA-4 | | 4 (11.4%) | 4 (22.2%) | 0 | |
| Chemother apy + anti-PD-1 | | 3 (8.6%) | 2 (11.1%) | 1 (5.9%) | |

Results are presented as numbers (%) or median [1st quartile - 3rd quartile]. The global functional status was assessed using the Eastern Cooperative Oncology Group Performance Status (ECOG-PS) scale, as described by Oken MM, Creech RH, Tormey DC, Horton J, Davis TE, McFadden ET, et al. Toxicity and response criteria of the Eastern Cooperative Oncology Group. Am J Clin Oncol. 1982;5:649-55 [18]. ECOG-PS was categorized into three levels (0-1, 2, and 3-4), where scores of 0 and 1 indicate fully active or restricted in strenuous activity to scores of 2 and higher indicating increasing disability. The comprehensive geriatric assessment included the following variables: autonomy (activities of daily living (ADL) score, abnormal if score ≤5/6; instrumental ADL (IADL) score, abnormal if score ≤7/8), mobility and fall risk (timed get up and go test, abnormal if unable to perform, or time to perform >20s), nutritional status (body mass index in kg/m$^2$ (BMI); absence or presence of weight loss ≥10% in the last 6 months), cognitive status (mini-mental state examination (MMSE), abnormal is score ≤24/30), mood (mini geriatric depression scale (GdS) score, risk for depression if score ≥1/4), and comorbidities (cumulative illness rating scale for geriatrics (CIRS-G), 5-point scale ranging from 0 (no dysfunction) to 4 (extremely severe dysfunction))) (Caillet P, Canoui-Poitrine F, Vouriot J, Berle M, Reinald N, Krypciak S, et al. Comprehensive Geriatric Assessment in the Decision-Making Process in Elderly Patients With Cancer: ELCAPA Study. JCO. 2011;29:3636-42 [16]).

ENT: ear nose and throat, BMI: Body Mass Index, Mini- GDS: Mini-Geriatric Depression Scale, MMSE Mini-Mental State Examination, TUG Time Up and Go, LDH: Lactate Dehydrogenase, NLR: neutrophil lymphocytes ratio, CRP: C reactive protein, ALBU: albumin, ICI: immune checkpoint inhibitor, NR: not relevant. Molecules: anti-PD1: pembrolizumab, nivolumab; anti-PD-L1: durvalumab, avelumab; chemotherapy prior to ICI: carboplatin and taxol, carboplatin and gemcitabin or taxol alone.

## 2. Senescence-associated CD8$^+$ T cell subsets in the cohort

CD8$^+$ T cell senescence phenotyping was performed by flow cytometry at baseline (i.e. prior to anti-PD-1/PD-L1 treatment). Among the 35 patients, data from 25 patients were eligible for dimensionality reduction and clustering and the 35 were eligible for conventional analysis.

### 2.1. Identification and prevalence of senescence-associated CD8$^+$ T cell subsets

The proportion of EMRA CD8$^+$ T cells varied among individuals, ranging from 2.8% to 38.2% with a mean ± SD of 13.7 ± 8.6 of the total CD8$^+$ T cells in the overall cohort. In this cohort of patients aged over 70 years old, the percentage of EMRA did not correlate significantly with age or sex ratio (data not shown). As shown in Figure 1, within the entire CD8$^+$ T cell population: (i) CD57 expression was predominantly expressed by CD28$^-$ cells (77.4% ± 17.7), and (ii) GRZB expression was observed mainly in CD28$^-$ cells (84.1 ± 17.6).

Unsupervised analysis using optSNE allowed the identification of CD8$^+$ T cell differentiation stages according to CD45RA, CD27, and CD28 expression. CD57 was mainly expressed by effector T cells (EM and EMRA). KLRG1 was mainly expressed by effector T cells (EM and EMRA), less so by CM T cells, and was weakly expressed, if at all, by naïve T cells (Figure 1). The expression of NKG2A among CD8$^+$ T cells was low, averaging around 5%. NKG2D and CD56 expressions were very weak, at less than 1% among the CD8$^+$ T cells in the cohort (Figure 6). Quantification of these markers as percentages among CD8$^+$ T cells is reported in Table 3.

**Table 3: Proportion of senescent-associated CD8+ T cell subsets among CD8+ T cell and among EMRA CD8+ T cell.** Results are presented in mean ± standard deviation (SD).

| Baseline percentage of cells | Mean ± SD |
|---|---|
| **Among CD8$^+$ T cells** | |
| CD28- | 50.8 ± 25.0 |
| CD57$^+$ | 52.1 ± 20.4 |
| CD57$^-$ | 47.9 ± 20.3 |
| KLRG1$^+$ | 66.1 ± 17.3 |
| KLRG1$^-$ | 33.9 ± 17.3 |
| CD57$^+$KLRG1$^+$ | 45.7 ± 20.0 |
| CD28$^-$CD57$^+$KLRG1$^+$ | 37.8 ± 21.9 |
| COS7$^+$KLRG1$^-$ | 54.3 ± 20.0 |
| CD56$^+$ | 0.7 ± 2.6 |
| NKG2A$^+$ | 5.2 ± 3.8 |
| NKG2D$^+$ | 0.3 ± 0.4 |
| GRZB$^+$ | 54.4 ± 23.2 |

(continued)

| Baseline percentage of cells | Mean ± SD |
|---|---|
| **Among CD8+ T cells** | |
| CD45RA+CD27-CD28- | 13.7 ± 8.6 |
| **Among Terminally differentiated EMRA CD8+ T cells (CD45RA+CD27-CD28-)** | |
| CD57+ | 80.5 ± 18.8 |
| CD57- | 19.5 ± 18.8 |
| KLRG1+ | 86.0 + 16.6 |
| KLRG1- | 14.0 ± 16.6 |
| CD57+KLRG1+ | 63.9 ± 16.7 |
| CD57+KLRG1- | 36.1 ± 16.7 |
| CD57+GRZB+ | 65.6 ± 16.7 |
| NKG2A+ | 2.3 ± 2.9 |
| GRZB+ | 86.1 ± 11.1 |

### 2.2. Characterization of EMRA CD8+ T cell

#### 2.2.1. Senescence markers

#### 2.2.1.1 NK receptors expression by EMRA CD8+ T cell

Among EMRA CD8+ T cells, the majority expressed KLRG1, with a mean ± SD of 86.0 ± 16.6%, compared to 66.1 ± 17.3% in the whole CD8+ T cell population (p<0.0001). Two clusters emerged based on CD57 expression: the predominant EMRA cluster was CD57+ (CD57 expression of 80.5 ± 18.8% *versus* 52.1 ± 20.4% in the whole CD8+ T cells, p<0.0001), while a smaller but significant subset of EMRA was CD57- (19.5 ± 18.8% *versus* 47.9 ± 20.3% in the whole CD8+ T cells, p<0.0001). EMRA CD8+ T cells were predominantly CD57+ KLRG1+ (63.9 ± 16.7% *versus* 45.7 ± 20.0% in the whole CD8+ T cells, p=0.0004). To note, a minor subset of CD57+ EMRA cells expressed high levels of the signaling adaptor molecule DAP-12, which is typically expressed in activated NK cells (Figure 1). The expression of NKG2A in EMRA CD8+ T cells averaged under 5%. Given the very low or absent expression of CD56 and NKG2D on CD8+ T cells in this cohort, their quantification on EMRA cells was not deemed relevant (Table 3).

#### 2.2.1.2 Sestrin expression

The stress-responsive protein sestrin-2, which accumulates with senescence, showed higher expression in more differentiated CD8+ T cell subsets compared to naïve cells (Figure 2 and Table 4: naïve *versus* CM : p=0.002, naïve *versus* EM: p=0.002, naïve *versus* EMRA: p=0.002).t. Its expression did not vary significantly between EMRA subsets according to CD57 expression (Figure 2, Table S3: p=ns). For each patient, the median value of all measurements performed on all cells belonging to the same differentiation stage (naive, CM, EM, EMRA, CD57-EMRA, CD57+EMRA, CD57+DAP-12+EMRA) was calculated to obtain the MFI (mean fluorescence intensity). The median MFI was determined in the 35 patients of the cohort.

### Table 4 Expression of sestrin-2, granzyme B and Ki-67 according to the differentiation stage

| Sestrin-2 | MFI | Granzyme B | MFI | Ki-67 | MFI |
|---|---|---|---|---|---|
| Naïve | 20174 | Naive | 2294 | Naive | 3411 |
| CM | 26609 | CM | 5849 | CM | 3500 |
| EM | 35310 | EM | 16176 | EM | 3377 |
| EMRA | 31270 | EMRA | 295788 | EMRA | 3476 |
| CD57-EMRA | 30302 | CD57- | 147223 | CD57- | 3430 |
| CD57+ | 31166 | CD57+ | 318116 | CD57+ | 3729 |

(continued)

| Sestrin-2 | MFI | Granzyme B | MFI | Ki-67 | MFI |
|---|---|---|---|---|---|
| CD57+DAP- | 33308 | CD57+ | 284830 | CD57+ | 3492 |

### 2.2.2. Granzyme B and Ki-67 expression

EMRA and CD57+ EMRA CD8+ T cells highly expressed GRZB, with means ± SD of 86.1 ± 11.1% and 80.7 ± 10.7% respectively. The more differentiated was the CD8+ T cell, the more it expressed GRZB, in particular, CD57+ EMRA expressed more GRZB compared to the CD57- EMRA CD8+ T cells (p<0.0001) (Figure 2). In contrast, Ki-67 expression did not vary significantly across CD8+ T cell differentiation stages (Figure 2).

### 3. CD8+ T cell senescence and its association with prior chemotherapy and anti-PD-1/PD-L1 treatment outcomes

### 3.1. Association with prior chemotherapy

CD57+ EMRA CD8+ T cells and its GRZB+ subset, but not total EMRA or CD57-EMRA or CD57+ or KLRG1+ CD8+ subsets, were significantly associated with prior chemotherapy treatment. In the group of patients who received chemotherapy before ICI, the median percentage among CD8+ T cells [IQR] of CD57+ EMRA and CD57+ GRZB+ EMRA CD8+ T cells were 12.5 [10.1 - 18.3] and 9.6 [8.6 - 13.8], respectively. These values were significantly higher compared to the chemotherapy-naive group, which had median values of 7.3 [3.9 - 10.3] and 5.8 [2.6 - 8.3], respectively (p=0.009 and p=0.007, Figure 3 A and Table 4). These associations remained significant after adjusting for sex and cancer type (Table 4).

Using a 10% threshold to define CD57+ EMRA CD8+ T cells positivity, 13 out of 17 patients (76.5%) in the pre-chemotherapy group were positive, compared to 6 out of 18 (33.3%) in the chemotherapy-naive group who received anti-PD-1/PD-L1 as a first-line treatment (p=0.010, adjusted p=0.013) (Figure 3B).

**Table 4 CD8+ T cell senescence according to previous exposure to chemotherapy**

| Baseline percentage among total CD8= T cell | Total (n=35) | Chemotherapy before ICI (n=17) | No chemotherapy before ICI (n=18) | *P-value* | *aP-value* |
|---|---|---|---|---|---|
| The table presents the median [IQR] proportion of the CD8+ T cells exhibiting senescence, stratified by prior exposure to chemotherapy. P-values are adjusted for cancer type (lung versus other) and sex. P-value<0.05 are highlighted in bold. | | | | | |
| **EMRA** | 12.6 [7.1 - 18.2] | 14.3 [11.5 - 18.5] | 8.5 [6.6 - 15.8] | 0.065 | 0.084 |
| **CD57+** | 55.2 [32.4 - 69.2] | 56.8 [41.1 - 74.4] | 54.4 [28.8 - 63.5] | 0.291 | 0.157 |
| **GRZB+** | 54.9 [35.8-77.8] | 56.3 [39.9 - 78.1] | 53.4 [24.2-69.7] | 0.409 | 0.191 |
| **EMRA KLRG1+** | 10.2 [6.7 - 14.3] | 11.7 [8.1 - 13.6] | 8 [5.9 - 14.3] | 0.106 | 0.199 |
| **EMRA CD57-** | 5.2 [2.7 - 6.9] | 4.2 [3.2 - 7.4] | 5.5 [2.7 - 6.4] | 0.921 | 0.734 |
| **EMRA CD57+** | 10.1 [6.3 - 14.6] | 12.5 [10.1 - 18.3] | 7.3 [3.9 - 10.3] | **0.009** | **0.014** |
| **EMRA GRZB+** | 9.8 [6.2 - 15.6] | 12.4 [9.7 - 17.3] | 7.2 [4.2 - 13.4] | **0.025** | **0.052** |
| **EMRA CD57 +GRZB+** | 8.3 [4.7 - 11.9] | 9.6 [8.6 - 13.8] | 5.8 [2.6 - 8.3] | **0.007** | **0.018** |

### 3.2. Association with response to anti-PD-1/PD-L1 treatment

When using a 10% threshold to define CD57+ GRZB+ EMRA CD8+ T cells positivity, 8/10 (80%) positive patients were non-responders (PD/Death), while only 2 out of 10 (20%) responders (SD/PR) (Figure 3C). A proportion of CD57+GRZB+EMRA CD8+ T cells ≥ 10% of the total CD8+ T cells population tended to be associated with non-response at six months (adjusted p=0.097, Table 5).

Using a 10% cutoff to define CD57+ EMRA CD8+ T cells positivity, 11/17 (64.7%) positive patients were non-responders, compared to 6 out of 17 (35.3%), responders (adjusted p=0.488, positive predictive value=64.7%) (Table 2 & Figure 3C). Other CD8+ T cells subsets, including the previously described Senescence Immunological Profile (SIP) i.e. CD28-CD57+KLRG1+ [21], did not allow discrimination between responder and non-responders to the anti-PD-1/PD-L1 therapy (Table 5).

**Table 5 CD8+ T cell senescence according to anti-PD-1/PD-L1 response**

| Percentage of the subset among total CD8+ T cell or number of patients (n (%)) with 10% positivity threshold | PD/Death | PR/SD | P-value | Multivariate logistic regression | |
| --- | --- | --- | --- | --- | --- |
| | N=18 | N=11 | | OR (IC 95%) | a P-value |
| **EMRA** (% among CD8) | 14 [7.6 - 18.5] | 11.5 [6.7-15.1] | 0.323 | 0.96 (0.86 ; 1.06) | 0.433 |
| n (%) (10% positivity threshold) | 12 (66.7) | 6 (54.5) | 0.696 | 0.38 (0.06 ; 2.39) | 0.306 |
| **CD57+** (% among CD8) | 57.8 [35.7 - 69.2] | 47.8 [31.0-70.7] | 0.621 | 0.99 (0.95 ; 1.03) | 0.603 |
| n (%) (10% positivity threshold) | 18 (100) | 11 (100) | - | - | - |
| **GRZB+** (% among CD8) | 58.9 [41.1 - 78.1] | 52.0 [28.4 - 69.7] | 0.418 | 0.99 (0.95 ; 1.02) | 0.408 |
| n (%) (10% positivity threshold) | 18 (100) | 11 (100) | - | - | - |
| **EMRA KLRG1+** (% among CD8) | 11.0 [7.2 - 17.2] | 8.8 [5.9 - 13.5] | 0.472 | 0.98 (0.88 ; 1.08) | 0.636 |
| n (%) (10% positivity threshold) | 10 (55.6) | 5 (45.5) | 0.597 | 0.44 (0.07 ; 2.70) | 0.372 |
| **EMRA CD57-** (% among CD8) | 5.6 [3.0 - 7.4] | 3.3 [2.1 - 6.4] | 0.418 | 1.00 (0.89 ; 1.13) | 0.974 |
| n (%) (10% positivity threshold) | 1 (5.6) | 1 (9.1) | >0.99 | 1.70 (0.16; 18.26) | 0.660 |
| **EMRA CD57+** (% among CD8) | 11.1 [6.3 - 16.2] | 10.1 [6.8 - 12.5] | 0.621 | 0.92 (0.80 ; 1.07) | 0.273 |
| n (%) (10% positivity threshold) | 11 (61.1) | 6 (54.5) | >0.99 | 0.54 (0.10 ; 3.05) | 0.488 |
| **EMRA GRZB+** (% among CD8) | 12.3 [6.8 - 16.0] | 9.4 [6.2 - 14.4] | 0.418 | 0.96 (0.86 ; 1.07) | 0.468 |
| n (%) (10% positivity threshold) | 11 (61.1) | 4 (36.4) | 0.196 | 0.15 (0.01 ; 1.56) | 0.112 |
| **EMRA CD57+GRZB+** (% among CD8) | 8.8 [4.3 - 12.3] | 7.3 [5.1 - 9.6] | 0.621 | 0.90 (0.76 ; 1.07) | 0.219 |
| n (%) (10% positivity threshold) | 8 (44.4) | 2 (18.2) | 0.234 | 0.18 (0.02 ; 1.37) | **0.097** |
| **CD28-KLRG1+CD57+** (% among CD8) | 38.0 [21.0 - 56.4] | 36.0 [20.1 - 58.1] | 0.753 | 0.99 (0.96 ; 1.03) | 0.732 |
| n (%) (10% positivity threshold) | 17 (94.4) | 10 (90.9) | >0.99 | 0.53 (0.04 ; 7.80) | 0.646 |

Table 5 displays the median [IQR] proportion of CD8+ T cell subsets, categorized by response to anti-PD-1/PD-L1 response. P-values are adjusted (aP-value) for chemotherapy pretreatment and cancer type (lung versus other). P-values between 0.05 and 0.1, which indicate a statistical trend, are shown in italic bold. Response categories are as follows: PD (progressive disease, including death within 6 months), PR (partial response), SD (stable disease).

| Table 6 Overall response rate and mortality according to EMRA CD57+ CD8+ T cell positivity Outcome | Available data | | EMRA CD57+ <10 n=16 | EMRA CD57+ ≥10 n=19 |
| --- | --- | --- | --- | --- |
| **ORR at 6 months** | 29 | | | |

(continued)

| Table 6 Overall response rate and mortality according to EMRA CD57+ CD8+ T cell positivity Outcome | Available data | | EMRA CD57+ <10 n=16 | EMRA CD57+ ≥10 n=19 |
|---|---|---|---|---|
| Complete response | | 0 | 0 | 0 |
| Partial response | | 6/29 (21%) | 3/12 (25%) | 3/17 (18%) |
| Stable disease | | 5/29 (17%) | 2/12 (17%) | 3/17 (18%) |
| Progressive disease | | 6/29 (21%) | 1/12 (8%) | 5/17 (29%) |
| Death | | 12/29 (41%) | 6/12 (50%) | 6/17 (35%) |
| | | | | |
| **Global death** | 35 | 23/35 | 11/17 | 12/17 |

The 6-month overall response rate and mortality are shown for the entire population and stratified by CD57+ EMRA positivity (using a 10% cut-off among CD8+ T cells).

Results Flow cytometry and unsupervised analysis were employed to characterize Effector Memory CD45RA+ (EMRA) and CD8+ T cell senescence at baseline, before initiating PD-1/PD-L1 therapy. EMRA cells were found to overexpress CD57 and KLRG1 compared to overall CD8+ T cells. Chemotherapy prior to anti-PD-1/PD-L1 was associated with an increased proportion of CD57+ EMRA CD8+ T cells (p=0.009) and its granzyme B (GRZB) subset (p=0.007). Using a 10% cut-off to define positivity, the six-month non-response tends to be associated with the CD57+ GRZB+ EMRA positivity (p=0.097). Other CD8+ T cell subsets (EMRA, CD57+, or KLRG1+), usually associated with senescence, showed no significant association with previous chemotherapy or response to anti-PD-1/anti-PD-L1 therapy.

Conclusions These findings underscore the impact of prior chemotherapy on expanding the pool of senescent T cells, particularly CD57+ EMRA CD8+ T and CD57+ GRZB+ EMRA CD8+ T cells, whose expansion could affect the effectiveness of anti-PD-1/PD-L1 immunotherapy in elderly patients. This highlights the need for tailored approaches in this population.

**Discussion**

The aim of the study was to characterize CD8+ T cell senescence in a cohort of patients aged over 70 years old receiving ICI and to study the association between prior chemotherapy exposure and cancer outcomes. We focused on EMRA T cells, NK-markers (CD57, KLRG1, CD56, NKG2D, Dap-12), and sestrin-2 expression, which are recognized markers of senescent CD8+ T cells. The percentage of EMRA among CD8+ T cells varied significantly between individuals, illustrating the inter-individual variability even after 70 years old. CD57 and KLRG-1 were mainly expressed on differentiated T cells, whereas CD56, NKG2A, and NKG2D were weakly expressed.

The more differentiated these CD8+ T cells were, the more they expressed Dap-12, a signaling adaptor typically found in NK cells. Notably, EMRA CD8+ T cells (i) overexpressed CD57 and KLRG1 compared to the overall CD8+ T cell population and (ii) were predominantly KLRG1+ and/or CD57+ suggesting that these highly differentiated T cells exhibit NK-like behavior in terms of NK receptors expression and signaling.

Two subsets of EMRA CD8+ T cell that emerged according to CD57 expression were identified: a predominant CD57+ subset and a smaller CD57- subset. Importantly, the CD57+ EMRA CD8+ T cell subset, but not total EMRA population or other subsets such as CD57+ or KLRG-1+, was associated with prior chemotherapy exposure. This support that chemotherapy induce T cell senescence, likely through mechanisms related to DNA damage. This finding aligns with previous studies showing reduced telomere length of T cells of NHL patients receiving chemotherapy [15,22]. Another study in a non-geriatric cohort demonstrated an increase in the proportion of the senescent subset CD28- CD57+ CD8+ T cells in lung cancer patients, with differential reconstitution of the CD8+ pool depending on CD28 and CD57 expression after chemotherapy (Onyema OO, Decoster L, Njemini R, Forti LN, Bautmans I, De Waele M, et al. Shifts in subsets of CD8+ T-cells as evidence of immunosenescence in patients with cancers affecting the lungs: an observational case-control study. BMC Cancer. 2015;15:1016. [23]). In the literature, Verma et al showed that, after stimulation, CD57+ EMRA CD8+ T cells are less prone to activation (decreased proliferation capacities and IFNy secretion) and had longer telomeres compared to the CD57- counterpart (Verma K, Ogonek J, Varanasi PR, Luther S, Bünting I, Thomay K, et al. Human CD8+ CD57- TEMRA cells: Too young to be called "old." PLoS One. 2017;12:e0177405. [28])

The overall response to anti-PD-1/PD-L1 therapy (stable disease and partial response versus progression or death) tended to be inversely associated with CD57+ GRZB+ EMRA CD8+ T cell positivity (p=0.097) was observed. Patients with progressive disease were more prone to exhibit CD57+ EMRA CD8+ T cell positivity. Previous studies have demonstrated that terminally differentiated CD8[+] T cells possess high functional and cytotoxic potential capacities and highly secrete the GRZB [21,24]. However, this GRZB expression is not necessarily linked to specific cytotoxicity. In a previous study, CD28[-] CD57[+] KLRG1[+] CD8[+] T cells (called Senescence Immunological Profile (SIP)) was associated with response to anti-PD-1/PD-L1 (using an optimal cut-off of 39.5% in the discovery cohort or 28% in the validation cohort), in an adult non-geriatric cohort [21]. However, in the present study of patients aged 70 and over, SIP was not significantly associated with the ORR. It is possible that CD57[+] EMRA subset is more relevant to this geriatric oncology population. The markers used in the SIP are closely related to those of CD57[+] EMRA (SIP: CD28[-] CD57[+] KLRG1[+] compared to CD57[+] EMRA: CD45RA[+] CD27[-] CD28[-] CD57[+]), reinforcing the relevance of CD57[+] and CD28[-] as key markers in the clinical characterization of T cell senescence.

CD57 expression by CD8[+] T cells is known to lead to replicative senescence and impaired proliferation. CD28[-] CD57[+] CD8[+] T cells have been characterized in the context of chronic antigen stimulation, such as HIV infection as well as chronic inflammation and cancer [25]. These cells may represent chronically stimulated cells with diminished functionality such as reduced perforin secretion [26]. Although EMRA CD8[+] T cells have high cytotoxic and cytokine secretion potential [24,27], they may have difficulties in activation and proliferation following specific stimulation [13]. One considers that they have a NK-like behaviour [12]. There are some differences in their characterisation, depending on the study.

This study is important as it is one of the first to focus on patients aged 70 and older. To our knowledge, this cohort is unique in the literature and addresses a clinically relevant question.

**Conclusions**

In summary, this example demonstrates, in particular in older cancer patients, that EMRA CD8[+] T cells can be subdivided into two subsets based on CD57 expression, with CD57[+] EMRA being predominant and associated with prior chemotherapy exposure. GRZB[+] $\pm$ CD57[+] EMRA subsets appear to be relevant markers to investigate in elderly patients receiving anti-PD-1/PD-L1 therapy, particularly following first-line chemotherapy. EMRA CD57[+] CD8[+] T cell is a marker of chemotherapy-induced senescence and (ii) first-line chemotherapy might be reconsidered before ICI in cases where it induces CD57[+] EMRA CD8[+] T cells.

The results also clearly demonstrate surprisingly that by accurately detecting and determining the ratio between CD57[+] EMRA CD8[+] T cells / CD8[+] T cells in a biological sample, that this ratio allows determining whether a subject suffers from immune ageing and/or immunosenescense. Advantageously, by accurately determining the ratio between CD57[+] EMRA CD8[+] T cells / CD8[+] T cells, this ratio significantly enhance clinical decision-making and improve patient outcomes.

The results also clearly demonstrate surprisingly that by accurately detecting and determining the ratio between CD57[+] EMRA CD8[+] T cells / CD8[+] T cells in a biological sample, that this ratio allows determining whether a subject suffers from immune ageing and/or immunosenescense and/or whether a condition or disease, preferably cancer, be sensitive to a therapeutic agent, in particular an immunotherapy.

Advantageously, by accurately determining the ratio between CD57[+] EMRA CD8[+] T cells / CD8[+] T cells, this ratio significantly enhance clinical decision-making and could improve patient outcomes.

**List of references**

1. Reck M, Rodriguez-Abreu D, Robinson AG, Hui R, Csöszi T, Fülöp A, et al. Pembrolizumab versus Chemotherapy for PD-L1-Positive Non-Small-Cell Lung Cancer. N Engl J Med. 2016;375:1823-33.

2. Subbiah V, Solit DB, Chan TA, Kurzrock R. The FDA approval of pembrolizumab for adult and pediatric patients with tumor mutational burden (TMB) ≥10: a decision centered on empowering patients and their physicians. Annals of Oncology. 2020;31:1115-8.

3. Beinse G, Reitter D, Segaux L, Carvahlo-Verlinde M, Rousseau B, Tournigand C, et al. Potential drug-drug interactions and risk of unplanned hospitalization in older patients with cancer: A survey of the prospective ELCAPA (ELderly CAncer PAtients) cohort. Journal of Geriatric Oncology. 2020;11:586-92.

4. Soubeyran P, Bellera C, Paillaud E. Achieving harmony in oncological geriatric assessment - Should we agree on a best set of tools? Journal of Geriatric Oncology. 2023;14:101482.

5. Pawelec G. Does patient age influence anti-cancer immunity? Semin Immunopathol. 2019;41:125-31.

6. Liu Z, Liang Q, Ren Y, Guo C, Ge X, Wang L, et al. Immunosenescence: molecular mechanisms and diseases. Sig Transduct Target Ther. 2023;8:1-16.

7. Gattinoni L, Speiser DE, Lichterfeld M, Bonini C. T memory stem cells in health and disease. Nat Med. 2017;23:18-27.

8. Koch S, Larbi A, Derhovanessian E, Ozcelik D, Naumova E, Pawelec G. Multiparameter flow cytometric analysis of CD4 and CD8 T cell subsets in young and old people. Immun Ageing. 2008;5:1-12.

9. Romero P, Zippelius A, Kurth I, Pittet MJ, Touvrey C, Iancu EM, et al. Four Functionally Distinct Populations of Human Effector-Memory CD8 + T Lymphocytes. J Immunol. 2007;178:4112-9.

10. Callender LA, Carroll EC, Bober EA, Akbar AN, Solito E, Henson SM. Mitochondrial mass governs the extent of human T cell senescence. Aging Cell. 2020;19:e13067.

11. Granier C, Gey A, Roncelin S, Weiss L, Paillaud E, Tartour E. Immunotherapy in older patients with cancer. Biomedical Journal. 2021;44:260-71.

12. Pereira BI, De Maeyer RPH, Covre LP, Nehar-Belaid D, Lanna A, Ward S, et al. Sestrins induce natural killer function in senescent-like CD8 + T cells. Nature Immunology. 2020;1-11.

13. Lanna A, Gomes DCO, Muller-Durovic B, McDonnell T, Escors D, Gilroy DW, et al. A sestrin-dependent Erk/Jnk/p38 MAPK activation complex inhibits immunity during ageing. Nat Immunol. 2017;18:354-63.

14. Hui E, Cheung J, Zhu J, Su X, Taylor MJ, Wallweber HA, et al. T cell costimulatory receptor CD28 is a primary target for PD-1-mediated inhibition. Science. 2017;355:1428-33.

15. Lee J-J, Nam C-E, Cho S-H, Park K-S, Chung I-J, Kim H-J. Telomere length shortening in non-Hodgkin's lymphoma patients undergoing chemotherapy. Ann Hematol. 2003;82:492-5.

16. Caillet P, Canoui-Poitrine F, Vouriot J, Berle M, Reinald N, Krypciak S, et al. Comprehensive Geriatric Assessment in the Decision-Making Process in Elderly Patients With Cancer: ELCAPA Study. JCO. 2011;29:3636-42.

17. Tazdait M, Mezquita L, Lahmar J, Ferrara R, Bidault F, Ammari S, et al. Patterns of responses in metastatic NSCLC during PD-1 or PDL-1 inhibitor therapy: Comparison of RECIST 1.1, irRECIST and iRECIST criteria. European Journal of Cancer. 2018;88:38-47.

18. Oken MM, Creech RH, Tormey DC, Horton J, Davis TE, McFadden ET, et al. Toxicity and response criteria of the Eastern Cooperative Oncology Group. Am J Clin Oncol. 1982;5:649-55.

19. Larbi A, Fulop T. From "truly naïve" to "exhausted senescent" T cells: When markers predict functionality. Cytometry Part A. 2014;85:25-35.

20. Belkina AC, Ciccolella CO, Anno R, Halpert R, Spidlen J, Snyder-Cappione JE. Automated optimized parameters for T-distributed stochastic neighbor embedding improve visualization and analysis of large datasets. Nat Commun. 2019;10:5415.

21. Ferrara R, Naigeon M, Auclin E, Duchemann B, Cassard L, Jouniaux JM, et al. Circulating T-cell immunosenescence in advanced non-small cell lung cancer patients treated with single agent PD-1/PD-L1 inhibitors or platinum-based chemotherapy. Clin Cancer Res. 2020;clincanres.1420.2020.

22. Li P, Hou M, Lou F, Björkholm M, Xu D. Telomere dysfunction induced by chemotherapeutic agents and radiation in normal human cells. The International Journal of Biochemistry & Cell Biology. 2012;44:1531-40.

23. Onyema OO, Decoster L, Njemini R, Forti LN, Bautmans I, De Waele M, et al. Shifts in subsets of CD8+ T-cells as evidence of immunosenescence in patients with cancers affecting the lungs: an observational case-control study. BMC Cancer. 2015;15:1016.

24. Seidel JA, Vukmanovic-Stejic M, Muller-Durovic B, Patel N, Fuentes-Duculan J, Henson SM, et al. Skin resident memory CD8+ T cells are phenotypically and functionally distinct from circulating populations and lack immediate cytotoxic function. Clin Exp Immunol. 2018;194:79-92.

25. Strioga M, Pasukoniene V, Characiejus D. CD8+ CD28- and CD8+ CD57+ T cells and their role in health and disease. Immunology. 2011;134:17-32.

26. Huff WX, Kwon JH, Henriquez M, Fetcko K, Dey M. The Evolving Role of CD8+CD28- Immunosenescent T Cells in Cancer Immunology. Int J Mol Sci. 2019;20:2810.

27. Henson SM, Lanna A, Riddell NE, Franzese O, Macaulay R, Griffiths SJ, et al. p38 signaling inhibits mTORC1-independent autophagy in senescent human CD8+ T cells [Internet]. American Society for Clinical Investigation; 2014 [cited 2022 Jun 27]. Available from: http://www.jci.org/articles/view/75051/pdf

28. Verma K, Ogonek J, Varanasi PR, Luther S, Bünting I, Thomay K, et al. Human CD8+ CD57- TEMRA cells: Too young to be called "old." PLoS One. 2017;12:e0177405.

29. Van Herck Y, Feyaerts A, Alibhai S, Papamichael D, Decoster L, Lambrechts Y, et al. Is cancer biology different in older patients? The Lancet Healthy Longevity. 2021;2:e663-77.

30. BONIFACINO, Juan S., CHEN, Catherine, LIPPINCOTT-SCHWARTZ, Jennifer, et al. Subunit interactions within the T-cell antigen receptor: clues from the study of partial complexes. Proceedings of the National Academy of Sciences, 1988, vol. 85, no 18, p. 6929-6933

31. Sun J, Kavathas PB. Comparison of the roles of CD8 alpha alpha and CD8 alpha beta in interaction with MHC class I. J Immunol. 1997 Dec 15;159(12):6077-82. PMID: 9550407

32. Raskov, H., Orhan, A., Christensen, J.P. et al. Cytotoxic CD8+ T cells in cancer and cancer immunotherapy. Br J Cancer 124, 359-367 (2021). https://doi.org/10.1038/s41416-020-01048-4

33. Kared H, Martelli S, Ng TP, Pender SL, Larbi A. CD57 in human natural killer cells and T-lymphocytes. Cancer Immunol Immunother. 2016 Apr;65(4):441-52. doi: 10.1007/s00262-016-1803-z. Epub 2016 Feb 5. PMID: 26850637; PMCID: PMC11029668

34. Kared, H., Martelli, S., Ng, T.P. et al. CD57 in human natural killer cells and T-lymphocytes. Cancer Immunol Immunother65, 441-452 (2016). https://doi-org.proxy.insermbiblio.inist.fr/10.1007/s00262-016-1803-z)

35. Koch, S., Larbi, A., Derhovanessian, E. et al. Multiparameter flow cytometric analysis of CD4 and CD8 T cell subsets in young and old people. Immun Ageing 5, 6 (2008). https://doi.org/10.1186/1742-4933-5-6

36. Romero P, Zippelius A, Kurth I, Pittet MJ, Touvrey C, Iancu EM, et al. Four Functionally Distinct Populations of Human Effector-Memory CD8 + T Lymphocytes. J Immunol. 2007;178:4112-9.

37. HINTZEN, Rogier Q., LENS, Susanne M., BECKMANN, M. Patricia, et al. Characterization of the human CD27 ligand, a novel member of the TNF gene family. Journal of immunology (Baltimore, Md.: 1950), 1994, vol. 152, no 4, p. 1762-1773.

38. Esensten JH, Helou YA, Chopra G, Weiss A, Bluestone JA. CD28 Costimulation: From Mechanism to Therapy. Immunity. 2016 May 17;44(5):973-88. doi: 10.1016/j.immuni.2016.04.020. PMID: 27192564; PMCID: PMC4932896

39. Ivetic A, Hoskins Green HL, Hart SJ. L-selectin: A Major Regulator of Leukocyte Adhesion, Migration and Signaling. Front Immunol. 2019 May 14;10:1068. doi: 10.3389/fimmu.2019.01068. PMID: 31139190; PMCID: PMC6527602.

40. Campbell JJ, Murphy KE, Kunkel EJ, Brightling CE, Soler D, Shen Z, Boisvert J, Greenberg HB, Vierra MA, Goodman SB, Genovese MC, Wardlaw AJ, Butcher EC, Wu L. CCR7 expression and memory T cell diversity in humans. J Immunol. 2001 Jan 15;166(2):877-84. doi: 10.4049/jimmunol.166.2.877. PMID: 11145663

41. Verma K, Ogonek J, Varanasi PR, Luther S, Bünting I, Thomay K, Behrens YL, Mischak-Weissinger E, Hambach L. Human CD8+ CD57- TEMRA cells: Too young to be called "old". PLoS One. 2017 May 8;12(5):e0177405. doi: 10.1371/journal.pone.0177405. PMID: 28481945; PMCID: PMC5421808

42. Mousset CM, Hobo W, Woestenenk R, Preijers F, Dolstra H, van der Waart AB. Comprehensive Phenotyping of T Cells Using Flow Cytometry. Cytometry A. 2019 Jun;95(6):647-654. doi: 10.1002/cyto.a.23724. Epub 2019 Feb 4. PMID: 30714682

43. Zacharias ZR, Houtman JCD. OMIP-099: 31-color spectral flow cytometry panel to investigate the steady-state phenotype of human T cells. Cytometry A. 2024 Jan;105(1):10-15. doi: 10.1002/cyto.a.24799. Epub 2023 Oct 9. PMID: 37814476; PMCID: PMC10842108

44. Cullen, S., Brunet, M. & Martin, S. Granzymes in cancer and immunity. Cell Death Differ 17, 616-623 (2010). https://doi.org/10.1038/cdd.2009.206

**Claims**

1. Method for determining from a biological sample the sensitivity of a condition, preferably cancer, to a therapeutic agent comprising:

   a) determining in a biological sample the amount of $CD57^+$ EMRA $CD8^+$ T cells and the amount of cells $CD8^+$ T cells

   b) calculating the percentage P1 of $CD57^+$ EMRA $CD8^+$ T cells / $CD8^+$ T cells in said biological sample according to the following formula

$$P1= (CD57^+ \text{ EMRA } CD8^+ \text{ T cells} / CD8^+ \text{ T cells})*100.$$

2. Method according to claim 1 wherein the biological sample is selected from the group comprising blood, biopsy, tissue biopsy and tissue section.

3. Method according to claim 1 or 2 wherein the therapeutic agent is immunotherapy, preferably an immunotherapy treatment with a PD-1 or PD-L1 antagonist.

4. Method according to any of claim 1 to 3, wherein the therapeutic agent is selected from the group comprising Pembrolizumab, Nivolumab and/or Atezolizumab.

5. Method according to any of claim 1 to 4, wherein the method further comprises

   c) determining in said biological sample the amount of $CD57^+$ $GRZB^+$ EMRA $CD8^+$ T cells and
   d) calculating the percentage P2 of $CD57^+$ $GRZB^+$ EMRA $CD8^+$ T cells / $CD8^+$ T cells in said biological sample according to the following formula

$$P2 = (CD57^+ \, GRZB^+ \, EMRA \, CD8^+ \, T \, cells \, / \, CD8^+ \, T \, cells)*100.$$

6. Method according to any one of claim 1 to 5, wherein the method is used for the detection of immune ageing and/or immunosenescence.

7. Method for detecting immune ageing and/or immunosenescense in a subject comprising:

   i. searching in a biological sample the amount of CD57$^+$ EMRA CD8$^+$ T cells and the amount of cells CD8$^+$ T cells
   ii. calculating the percentage P3 of CD57$^+$ EMRA CD8$^+$ T cells / CD8$^+$ T cells in said biological sample according to the following formula

$$P3 = (CD57^+ \, EMRA \, CD8^+ \, T \, cells \, / \, CD8^+ \, T \, cells)*100.$$

8. Method according to claim 7, wherein the method further comprises

   iii. searching in said biological sample the amount of CD57$^+$ GRZB$^+$ EMRA CD8$^+$ T cells and
   iv. calculating the percentage P4 of CD57$^+$ GRZB$^+$ EMRA CD8$^+$ T cells / CD8$^+$ T cells in said biological sample according to the following formula
   P4= (CD57$^+$ GRZB$^+$ EMRA CD8$^+$ T cells / CD8$^+$ T cells)*100 .

9. A device for implementing a method according to any one of claims 1 to 8, comprising at least one mean for detecting and/or quantifying CD8$^+$T cells, at least one mean for detecting and/or quantifying CD57$^+$ EMRA CD8$^+$ T cells, and optionally at least one mean for detecting and/or quantifying CD57+ GRZB+ EMRA CD8+ T cells.

10. Kit for implementing a method according to any one of claims 1 to 8 comprising at least one detecting agent for detecting and/or quantifying CD8$^+$ T cells and at least one detecting agent for detecting and/or quantifying CD57$^+$ EMRA CD8$^+$ T cells.

11. Kit according to claim 10 wherein the kit comprises at least one detecting agent for detecting and/or quantifying CD57$^+$ GRZB$^+$ EMRA CD8$^+$ T cells.

EP 4 749 280 A1

A

B

FIGURE 1 (1/4)

30

C

D

FIGURE 1 (2/4)

E

F

FIGURE 1 (3/4)

G

KLRG1

H

25 files overlaid

CD57⁻ EMRA

CM

EM

Naïve

CD57⁺ EMRA

CD57⁻ EMRA

EM

FIGURE 1

FIGURE 2

FIGURE 3

A

B

FIGURE 4 (1/3)

C

D

FIGURE 4 (2/3)

E

**Terminally differentiated EMRA**

(Scatter plot: KLRG1 vs CD57)

F

Terminally differentiated EMRA    Terminally differentiated EMRA    Terminally differentiated EMRA

NKG2A          CD56          NKG2D

NKG2A          CD56          NKG2D

FIGURE 4 (3/3)

Number at risk (events)   35  (12)  21   (4)   17   (4)   10   (2)   6   (0)   3   (1)   1   (0)   1   (0)   0

FIGURE 5

A

FIGURE 6 (1/2)

B

FIGURE 6 (2/2)

Europäisches
Patentamt

European
Patent Office

Office européen
des brevets

# EUROPEAN SEARCH REPORT

**Application Number**

EP 25 17 2340

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | FEHLINGS MICHAEL ET AL: "Single-cell analysis reveals clonally expanded tumor-associated CD57 + CD8 T cells are enriched in the periphery of patients with metastatic urothelial cancer responding to PD-L1 blockade", JOURNAL FOR IMMUNOTHERAPY OF CANCER, vol. 10, no. 8, 14 June 2022 (2022-06-14), XP093309221, GB ISSN: 2051-1426, DOI: 10.1136/jitc-2022-004759 Retrieved from the Internet: URL:https://pmc.ncbi.nlm.nih.gov/articles/PMC9394212/pdf/jitc-2022-004759.pdf> * figures 3,4 * | 1-6,9-11 | INV. G01N33/50 G01N33/569 G01N33/574 |
| X | & FEHLINGS MICHAEL ET AL: "Supplementary Methods - Single-cell analysis reveals clonally expanded tumor-associated CD57 + CD8 T cells are enriched in the periphery of patients with metastatic urothelial cancer responding to PD-L1 blockade", JOURNAL FOR IMMUNOTHERAPY OF CANCER, vol. 10, no. 8, 14 June 2022 (2022-06-14), XP093310075, GB ISSN: 2051-1426, DOI: 10.1136/jitc-2022-004759 Retrieved from the Internet: URL:https://jitc.bmj.com/content/jitc/suppl/2022/08/18/jitc-2022-004759.DC1/jitc-2022-004759supp001_data_supplement.pdf> * figure S6 * | 1-6,9-11 | |

-----

-/--

**TECHNICAL FIELDS SEARCHED (IPC)**

G01N

The present search report has been drawn up for all claims

| Place of search | Date of completion of the search | Examiner |
|---|---|---|
| The Hague | 2 September 2025 | Wiesner, Martina |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

................................................................................

& : member of the same patent family, corresponding document

EPO FORM 1503 03.82 (P04C01)

page 1 of 3

| | Europäisches Patentamt |
| --- | --- |
| | European Patent Office |
| | Office européen des brevets |

## EUROPEAN SEARCH REPORT

**Application Number**

EP 25 17 2340

### DOCUMENTS CONSIDERED TO BE RELEVANT

| Category | Citation of document with indication, where appropriate, of relevant passages | Relevant to claim | CLASSIFICATION OF THE APPLICATION (IPC) |
| --- | --- | --- | --- |
| X | KINNEY BRENDAN L.C. ET AL: "CD28-CD57+ T cells from head and neck cancer patients produce high levels of cytotoxic granules and type II interferon but are not senescent", ONCOIMMUNOLOGY, vol. 13, no. 1, 14 June 2024 (2024-06-14), XP093309617, United States ISSN: 2162-402X, DOI: 10.1080/2162402X.2024.2367777 Retrieved from the Internet: URL:https://www.tandfonline.com/doi/pdf/10.1080/2162402X.2024.2367777> | 9-11 | |
| Y | * page 2, right-hand column, paragraph 5 - page 3, left-hand column, paragraph 1 * * abstract; figure 4 * * page 5 - page 6, left-hand column * ----- | 5-8 | |
| X | SARA DE BIASI ET AL: "Redistribution of CD8+ T cell subsets in metastatic renal cell carcinoma patients treated with anti-PD-1 therapy", CYTOMETRY A, WILEY-LISS, HOBOKEN, USA, vol. 101, no. 7, 4 May 2022 (2022-05-04), pages 597-605, XP072503618, ISSN: 1552-4922, DOI: 10.1002/CYTO.A.24562 | 9-11 | TECHNICAL FIELDS SEARCHED (IPC) |
| Y | * page 598, right-hand column, paragraph 4 - page 599, right-hand column, paragraph 2; figure 1 * ----- -/-- | 5,6 | |

The present search report has been drawn up for all claims

| Place of search | Date of completion of the search | Examiner |
| --- | --- | --- |
| The Hague | 2 September 2025 | Wiesner, Martina |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

........................................................................................

& : member of the same patent family, corresponding document

EPO FORM 1503 03.82 (P04C01)

**page 2 of 3**

Europäisches Patentamt

European Patent Office

Office européen des brevets

# EUROPEAN SEARCH REPORT

**Application Number**

**EP 25 17 2340**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | VERMA KRITI ET AL: "Human CD8+ CD57-TEMRA cells: Too young to be called "old"", PLOS ONE, vol. 12, no. 5, 8 May 2017 (2017-05-08), page e0177405, XP093019941, DOI: 10.1371/journal.pone.0177405 | 9-11 | |
| Y | * page 3, paragraph 2; figure 1; table 1 * | 5,6 | |
| Y | WO 2019/215740 A1 (TECHNION RES & DEV FOUNDATION [IL]) 14 November 2019 (2019-11-14) * claims 1,2 * | 7,8 | |
| | | | TECHNICAL FIELDS SEARCHED (IPC) |

The present search report has been drawn up for all claims

| Place of search | Date of completion of the search | Examiner |
|---|---|---|
| The Hague | 2 September 2025 | Wiesner, Martina |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 25 17 2340

This annex lists the patent family members relating to the patent documents cited in the above-mentioned European search report.
The members are as contained in the European Patent Office EDP file on
The European Patent Office is in no way liable for these particulars which are merely given for the purpose of information.

02-09-2025

| Patent document cited in search report | Publication date | Patent family member(s) | Publication date |
|---|---|---|---|
| WO 2019215740 A1 | 14-11-2019 | EP 3791181 A1 | 17-03-2021 |
| | | US 2021287807 A1 | 16-09-2021 |
| | | WO 2019215740 A1 | 14-11-2019 |

EPO FORM P0459

For more details about this annex : see Official Journal of the European Patent Office, No. 12/82

## REFERENCES CITED IN THE DESCRIPTION

**Non-patent literature cited in the description**

- **SUBBIAH V** ; **SOLIT DB** ; **CHAN TA** ; **KURZROCK R**. The FDA approval of pembrolizumab for adult and pediatric patients with tumor mutational burden (TMB) ≥10: a decision centered on empowering patients and their physicians. *Annals of Oncology.*, 2020, vol. 31, 1115-8
- **BEINSE G** ; **REITTER D** ; **SEGAUX L** ; **CARVAHLO-VERLINDE M** ; **ROUSSEAU B** ; **TOURNIGAND C et al.** Potential drug-drug interactions and risk of unplanned hospitalization in older patients with cancer: A survey of the prospective ELCAPA (ELderly CAncer PAtients) cohort. *Journal of Geriatric Oncology*, 2020, vol. 11, 586-92
- **SOUBEYRAN P** ; **BELLERA C** ; **PAILLAUD E**. Achieving harmony in oncological geriatric assessment - Should we agree on a best set of tools?. *Journal of Geriatric Oncology*, 2023, vol. 14, 101482
- **PAWELEC G.** Does patient age influence anti-cancer immunity?. *Semin Immunopathl.*, 2019, vol. 41, 125-31
- **LIU Z** ; **LIANG Q** ; **REN Y** ; **GUO C** ; **GE X** ; **WANG L et al.** Immunosenescence: molecular mechanisms and diseases. *Sig Transduct Target Ther.*, 2023, vol. 8, 1-16
- **GATTINONI L** ; **SPEISER DE** ; **LICHTERFELD M** ; **BONINI C.** T memory stem cells in health and disease. *Nat Med.*, 2017, vol. 23, 18-27
- **KOCH S** ; **LARBI A** ; **DERHOVANESSIAN E** ; **OZCELIK D** ; **NAUMOVA E** ; **PAWELEC G**. Multiparameter flow cytometric analysis of CD4 and CD8 T cell subsets in young and old people. *Immun Ageing*, 2008, vol. 5, 1-12
- **ROMERO P** ; **ZIPPELIUS A** ; **KURTH I** ; **PITTET MJ** ; **TOUVREY C** ; **LANCU EM et al.** Four Functionally Distinct Populations of Human Effector-Memory CD8 + T Lymphocytes. *J Immunol.*, 2007, vol. 178, 4112-9
- **CALLENDER LA** ; **CARROLL EC** ; **BOBER EA** ; **AKBAR AN** ; **SOLITO E** ; **HENSON SM**. Mitochondrial mass governs the extent of human T cell senescence. *Aging Cell*, 2020, vol. 19, e13067
- **GRANIER C** ; **GEY A** ; **RONCELIN S** ; **WEISS L** ; **PAILLAUD E** ; **TARTOUR E.** Immunotherapy in older patients with cancer. *Biomedical Journal*, 2021, vol. 44, 260-71
- **PEREIRA BI** ; **DE MAEYER RPH** ; **COVRE LP** ; **NEHAR-BELAID D** ; **LANNA A** ; **WARD S et al.** Sestrins induce natural killer function in senescent-like CD8 + T cells. *Nature Immunology*, 2020, 1-11
- **LANNA A** ; **GOMES DCO** ; **MULLER-DUROVIC B** ; **MCDONNELL T** ; **ESCORS D** ; **GILROY DW et al.** A sestrin-dependent Erk/Jnk/p38 MAPK activation complex inhibits immunity during ageing. *Nat Immunol.*, 2017, vol. 18, 354-63
- **LEE J-J** ; **NAM C-E** ; **CHO S-H** ; **PARK K-S** ; **CHUNG I-J** ; **KIM H-J**. Telomere length shortening in non-Hodgkin's lymphoma patients undergoing chemotherapy. *Ann Hematol.*, 2003, vol. 82, 492-5
- **BONIFACINO, JUAN S.** ; **CHEN, CATHERINE** ; **LIPPINCOTT-SCHWARTZ, JENNIFER et al.** Subunit interactions within the T-cell antigen receptor: clues from the study of partial complexes. *Proceedings of the National Academy of Sciences*, 1988, vol. 85 (18), 6929-6933
- **SUN J** ; **KAVATHAS PB**. Comparison of the roles of CD8 alpha alpha and CD8 alpha beta in interaction with MHC class I. *J Immunol.*, 15 December 1997, vol. 159 (12), 6077-82
- **RASKOV, H.** ; **ORHAN, A.** ; **CHRISTENSEN, J.P. et al.** Cytotoxic CD8+ T cells in cancer and cancer immunotherapy. *Br J Cancer*, 2021, vol. 124, 359-367, https://doi.org/10.1038/s41416-020-01048-4
- **KARED H** ; **MARTELLI S** ; **NG TP** ; **PENDER SL** ; **LARBI A**. CD57 in human natural killer cells and T-lymphocytes. *Cancer Immunol Immunother.*, 05 February 2016, vol. 65 (4), 441-52
- **KARED, H.** ; **MARTELLI, S.** ; **NG, T.P. et al.** CD57 in human natural killer cells and T-lymphocytes. *Cancer Immunol Immunother*, 2016, vol. 65, 441-452, https://doi-org.proxy.insermbiblio.inist.fr/10.1007/s00262-016-1803-z
- **KOCH, S.** ; **LARBI, A.** ; **DERHOVANESSIAN, E. et al.** Multiparameter flow cytometric analysis of CD4 and CD8 T cell subsets in young and old people. *Immun Ageing*, 2008, vol. 5, 6, https://doi.org/10.1186/1742-4933-5-6
- **ROMERO P** ; **ZIPPELIUS A** ; **KURTH I** ; **PITTET MJ** ; **TOUVREY C** ; **LANCU EM et al.** Four Functionally Distinct Populations of Human Effector-Memory CD8 T Lymphocytes. *J Immunol.*, 2007, vol. 178, 4112-9

- **HINTZEN, ROGIER Q.** ; **LENS, SUSANNE M.** ; **BECKMANN, M. PATRICIA et al.** Characterization of the human CD27 ligand, a novel member of the TNF gene family. *Journal of immunology (Baltimore, Md.: 1950)*, 1994, vol. 152 (4), 1762-1773
- **ESENSTEN JH** ; **HELOU YA** ; **CHOPRA G** ; **WEISS A** ; **BLUESTONE JA.** CD28 Costimulation: From Mechanism to Therapy. *Immunity*, 17 May 2016, vol. 44 (5), 973-88
- **IVETIC A** ; **HOSKINS GREEN HL** ; **HART SJ.** L-selectin: A Major Regulator of Leukocyte Adhesion, Migration and Signaling. *Front Immunol.*, 14 May 2019, vol. 10, 1068
- **CAMPBELL JJ** ; **MURPHY KE** ; **KUNKEL EJ** ; **BRIGHTLING CE** ; **SOLER D** ; **SHEN Z** ; **BOISVERT J** ; **GREENBERG HB** ; **VIERRA MA** ; **GOODMAN SB.** CCR7 expression and memory T cell diversity in humans. *J Immunol.*, 15 January 2001, vol. 166 (2), 877-84
- **VERMA K** ; **OGONEK J** ; **VARANASI PR** ; **LUTHER S** ; **BÜNTING I** ; **THOMAY K** ; **BEHRENS YL** ; **MISCHAK-WEISSINGER E** ; **HAMBACH L.** Human CD8+ CD57- TEMRA cells: Too young to be called ''old. *PLoS One*, 08 May 2017, vol. 12 (5), e0177405
- **MOUSSET CM** ; **HOBO W** ; **WOESTENENK R** ; **PREIJERS F** ; **DOLSTRA H** ; **VAN DER WAART AB.** Comprehensive Phenotyping of T Cells Using Flow Cytometry. *Cytometry A.*, 04 February 2019, vol. 95 (6), 647-654
- **ZACHARIAS ZR** ; **HOUTMAN JCD.** OMIP-099: 31-color spectral flow cytometry panel to investigate the steady-state phenotype of human T cells. *Cytometry A*, 09 October 2023, vol. 105 (1), 10-15
- **CULLEN, S.** ; **BRUNET, M.** ; **MARTIN, S.** Granzymes in cancer and immunity. *Cell Death Differ*, 2010, vol. 17, 616-623, https://doi.org/10.1038/cdd.2009.206
- **LIU, Z** ; **LIANG, Q.** ; **REN, Y. et al.** Immunosenescence: molecular mechanisms and diseases. *Sig Transduct Target Ther*, 2023, vol. 8, 200, https://doi.org/10.1038/s41392-023-01451-2
- **GRANIER, C.** ; **GEY, A.** ; **RONCELIN, S.** ; **WEISS, L.** ; **PAILLAUD, E.** ; **TARTOUR, E.** Immunotherapy in older patients with cancer. *biomedical journal*, 2021, vol. 44 (3), 260-271
- **LIU, Z.** ; **LIANG, Q.** ; **REN, Y. et al.** Immunosenescence: molecular mechanisms and diseases. *Sig Transduct Target Ther*, 2023, vol. 8, 200, https://doi.org/10.1038/s41392-023-01451-2
- **GRANIER, C.** ; **GEY, A** ; **RONCELIN, S.** ; **WEISS, L.** ; **PAILLAUD, E.** ; **TARTOUR, E.** Immunotherapy in older patients with cancer. *biomedical journal*, 2021, vol. 44 (3), 260-271
- **CAILLET P** ; **CANOUI-POITRINE F** ; **VOURIOT J** ; **BERLE M** ; **REINALD N** ; **KRYPCIAK S et al.** Comprehensive Geriatric Assessment in the Decision-Making Process in Elderly Patients With Cancer: ELCAPA Study. *JCO*, 2011, vol. 29, 3636-42
- **TAZDAIT M** ; **MEZQUITA L** ; **LAHMAR J** ; **FERRARA R** ; **BIDAULT F** ; **AMMARI S et al.** Patterns of responses in metastatic NSCLC during PD-1 or PDL-1 inhibitor therapy: Comparison of RECIST 1.1, irRECIST and iRECIST criteria. *European Journal of Cancer*, 2018, vol. 88, 38-47
- **KOCH S** ; **LARBI A** ; **DERHOVANESSIAN E** ; **OZCELIK D** ; **NAUMOVA E** ; **PAWELEC G.** Multi-parameter flow cytometric analysis of CD4 and CD8 T cell subsets in young and old people. *Immun Ageing.*, 2008, vol. 5, 1-12
- **BELKINA AC** ; **CICCOLELLA CO** ; **ANNO R** ; **HALPERT R** ; **SPIDLEN J** ; **SNYDER-CAPPIONE JE.** Automated optimized parameters for T-distributed stochastic neighbor embedding improve visualization and analysis of large datasets. *Nat Commun.*, 2019, vol. 10, 5415
- **OKEN MM** ; **CREECH RH** ; **TORMEY DC** ; **HORTON J** ; **DAVIS TE** ; **MCFADDEN ET et al.** Toxicity and response criteria of the Eastern Cooperative Oncology Group. *Am J Clin Oncol.*, 1982, vol. 5, 649-55
- **CAILLET P** ; **CANOUI-POITRINE F** ; **VOURIOT J** ; **BERLE M** ; **REINALD N** ; **KRYPCIAK S et al.** Comprehensive Geriatric Assessment in the Decision-Making Process in Elderly Patients With Cancer. *ELCAPA Study. JCO*, 2011, vol. 29, 3636-42
- **ONYEMA OO** ; **DECOSTER L** ; **NJEMINI R** ; **FORTI LN** ; **BAUTMANS I** ; **DE WAELE M et al.** Shifts in subsets of CD8+ T-cells as evidence of immunosenescence in patients with cancers affecting the lungs: an observational case-control study. *BMC Cancer.*, 2015, vol. 15, 1016
- **VERMA K** ; **OGONEK J** ; **VARANASI PR** ; **LUTHER S** ; **BÜNTING I** ; **THOMAY K et al.** Human CD8+ CD57- TEMRA cells: Too young to be called ''old.. *PLoS One*, 2017, vol. 12, e0177405
- **RECK M** ; **RODRIGUEZ-ABREU D** ; **ROBINSON AG** ; **HUI R** ; **CSÖSZI T** ; **FÜLÖP A et al.** Pembrolizumab versus Chemotherapy for PD-L1-Positive Non-Small-Cell Lung Cancer. *N Engl J Med.*, 2016, vol. 375, 1823-33
- **SUBBIAH V** ; **SOLIT DB** ; **CHAN TA** ; **KURZROCK R.** The FDA approval of pembrolizumab for adult and pediatric patients with tumor mutational burden (TMB) ≥10: a decision centered on empowering patients and their physicians. *Annals of Oncology*, 2020, vol. 31, 1115-8

- **KOCH S** ; **LARBI A** ; **DERHOVANESSIAN E** ; **OZCELIK D** ; **NAUMOVA E** ; **PAWELEC G**. Multi-parameter flow cytometric analysis of CD4 and CD8 T cell subsets in young and old people. *Immun Ageing.*, 2008, vol. 5, 1-12
- **GRANIER C** ; **GEY A** ; **RONCELIN S** ; **WEISS L** ; **PAILLAUD E** ; **TARTOUR E**. Immunotherapy in older patients with cancer. *Biomedical Journal*, 2021, vol. 44, 260-71
- **HUI E** ; **CHEUNG J** ; **ZHU J** ; **SU X** ; **TAYLOR MJ** ; **WALLWEBER HA et al.** T cell costimulatory receptor CD28 is a primary target for PD-1-mediated inhibition. *Science*, 2017, vol. 355, 1428-33
- **LARBI A** ; **FULOP T**. From "truly naïve" to "exhausted senescent" T cells: When markers predict functionality. *Cytometry Part A.*, 2014, vol. 85, 25-35
- **FERRARA R** ; **NAIGEON M** ; **AUCLIN E** ; **DUCH-EMANN B** ; **CASSARD L** ; **JOUNIAUX JM et al.** Circulating T-cell immunosenescence in advanced non-small cell lung cancer patients treated with single agent PD-1/PD-L1 inhibitors or platinum-based chemotherapy. *Clin Cancer Res.*, 2020
- **LI P** ; **HOU M** ; **LOU F** ; **BJÖRKHOLM M** ; **XU D**. Telomere dysfunction induced by chemotherapeutic agents and radiation in normal human cells. *The International Journal of Biochemistry & Cell Biology*, 2012, vol. 44, 1531-40
- **ONYEMA OO** ; **DECOSTER L** ; **NJEMINI R** ; **FORTI LN** ; **BAUTMANS I** ; **DE WAELE M et al.** Shifts in subsets of CD8+ T-cells as evidence of immunosenescence in patients with cancers affecting the lungs: an observational case-control study. *BMC Cancer*, 2015, vol. 15, 1016
- **SEIDEL JA** ; **VUKMANOVIC-STEJIC M** ; **MULLER-DUROVIC B** ; **PATEL N** ; **FUENTES-DUCULAN J** ; **HENSON SM et al.** Skin resident memory CD8+ T cells are phenotypically and functionally distinct from circulating populations and lack immediate cytotoxic function. *Clin Exp Immunol.*, 2018, vol. 194, 79-92
- **STRIOGA M** ; **PASUKONIENE V** ; **CHARACIEJUS D**. CD8+ CD28- and CD8+ CD57+ T cells and their role in health and disease. *Immunology*, 2011, vol. 134, 17-32
- **HUFF WX** ; **KWON JH** ; **HENRIQUEZ M** ; **FETCKO K** ; **DEY M**. The Evolving Role of CD8+CD28-Immunosenescent T Cells in Cancer Immunology. *Int J Mol Sci.*, 2019, vol. 20, 2810
- **HENSON SM** ; **LANNA A** ; **RIDDELL NE** ; **FRANZ-ESE O** ; **MACAULAY R** ; **GRIFFITHS SJ et al.** p38 signaling inhibits mTORC1-independent autophagy in senescent human CD8+ T cells. American Society for Clinical Investigation, 2014
- **VAN HERCK Y** ; **FEYAERTS A** ; **ALIBHAI S** ; **PAPAMICHAEL D** ; **DECOSTER L** ; **LAMBRECHTS Y et al.** Is cancer biology different in older patients?. *The Lancet Healthy Longevity*, 2021, vol. 2, e663-77
- **KARED, H** ; **MARTELLI, S.** ; **NG, T.P. et al.** CD57 in human natural killer cells and T-lymphocytes. *Cancer Immunol Immunother*, 2016, vol. 65, 441-452, https://doi-org.proxy.insermbiblio.inist.fr/10.1007/s00262-016-1803-z
- **KOCH, S.** ; **LARBI, A** ; **DERHOVANESSIAN, E. et al.** Multiparameter flow cytometric analysis of CD4 and CD8 T cell subsets in young and old people. *Immun Ageing*, 2008, vol. 5, 6, https://doi.org/10.1186/1742-4933-5-6
- **ZACHARIAS ZR** ; **HOUTMAN JCD**. OMIP-099: 31-color spectral flow cytometry panel to investigate the steady-state phenotype of human T cells. *Cytometry A.*, 09 October 2023, vol. 105 (1), 10-15